# EUROPEAN PATENT SPECIFICATION

(11) **EP 3 237 451 B1**
(45) Date of publication and mention of the grant of the patent: **24.07.2019**
(21) Application number: 15823810.5
(22) Date of filing: 23.12.2015
(51) Int. Cl.: C07K 16/30, G01N 33/574

(54) **PRO-APOPTOTIC ANTI-NG2/CSPG4 ANTIBODIES AND THEIR USES FOR DISEASE THERAPY**
PRO-APOPTOTISCHE ANTI-NG2/CSPG4-ANTIKÖRPER UND DEREN VERWENDUNGEN ZUR KRANKHEITSTHERAPIE
ANTICORPS ANTI-NG2/CSPG4 PRO-APOPTOTIQUES ET LEURS UTILISATIONS POUR LE TRAITEMENT DE MALADIES

(30) Priority: 23.12.2014 IT MI20142225; 23.12.2014 US 201462095836 P
(43) Date of publication of application: 01.11.2017
(73) Proprietor: Università degli Studi di Parma, 43121 Parma (IT)
(72) Inventor: PERRIS, Roberto, 20131 Milano (IT); DALLATOMASINA, Alice, 20127 Milano (IT); NICOLOSI, Pier Andrea, 33084 Cordenos (Pordenone) (IT); TAMBURINI, Elisa, 43125 Parma (IT); MUCIGNAT, Maria Teresa, 33081 Aviano (Pordenone) (IT)
(74) Representative: Ferreccio, Rinaldo
(86) International application number: PCT/IB2015/059921
(87) International publication number: WO 2016/103205

(56) References cited:
- WO-A1-2012/075324
- WO-A2-2010/033866
- US-A1- 2014 242 079
- ALICE DALLATOMASINA: "STRUCTURAL AND FUNCTIONAL TRAITS OF NG2/CSPG4 PROTEOGLYCAN IN RELATION TO ITS ROLE IN TUMORS", UNIVERSITÀ DEGLI STUDI DI PARMA , 2013, pages FP-95, XP002756001, Retrieved from the Internet: URL:http://dspace-unipr.cineca.it/bitstrea m/1889/2186/1/Tesi%20PhD%20Alice%20Dallato masina%20XXV%20ciclo%20COMPLETA.pdf [retrieved on 2016-04-01]
- "Tesi PhD Alice Dallatomasina XXV ciclo COMPLETA.pdf Tesi di Dottorato 5,39 MB Adobe PDF (STRUCTURAL AND FUNCTIONAL TRAITS OF NG2/CSPG4 PROTEOGLYCAN IN RELATION TO ITS ROLE IN TUMORS)", DSpaceUniPr , 2013, page 1, XP002756002, Retrieved from the Internet: URL:http://dspace-unipr.cineca.it/handle/1 889/2186 [retrieved on 2016-04-01]
- GIROLAMO FRANCESCO ET AL: "Diversified expression of NG2/CSPG4 isoforms in glioblastoma and human foetal brain identifies pericyte subsets.", PLOS ONE, vol. 8, no. 12, E84883, December 2013 (2013-12), pages 1-19, XP002756003, ISSN: 1932-6203

### SEQUENCE LISTING

<110> UNIVERSITA' DEGLI STUDI DI PARMA
<120> Anti-NG2/CSPG4 pro-apoptotic antibodies and their uses for desease therapy
<130> 21.U0023A.12.WO.1
<160> 39
<170> PatentIn version 3.5
<210> 1
   <211> 112
   <212> PRT
   <213> Artificial Sequence
<220>
   <223> Heavy chain variable region sequence of 2161D2 clone antibody (protein)
<400> 1
   Gln Val Lys Leu Glu Gln Ser Gly Gly Gly Leu Val Gln Pro Gly Gly
   1               5                   10                  15
   Ser Arg Lys Leu Ser Cys Ala Ala Ser Gly Phe Thr Phe Ser Ser Phe
               20                  25                  30
   Gly Met His Trp Val Arg Gln Ala Pro Glu Lys Gly Leu Glu Trp Val
           35                  40                  45
   Ala Tyr Ile Ser Ser Gly Ser Ser Thr Leu His Tyr Ala Asp Thr Val
       50                  55                  60
   Lys Gly Arg Phe Thr Ile Ser Arg Asp Asn Pro Lys Asn Thr Leu Phe
   65                  70                  75                      80
   Leu Gln Met Lys Leu Pro Ser Leu Cys Tyr Gly Leu Leu Gly Ser Arg
                   85                  90                  95
   Asp Leu Ser His Arg Leu Leu Ser Gln Asn Asp Thr Pro Ile Cys Leu
               100                 105                 110
<210> 2
   <211> 336
   <212> DNA
   <213> Artificial Sequence
<220>
   <223> Heavy chain variable region sequence of 2161D2 clone antibody (DNA)
<400> 2
   caggtcaagc tggagcagtc tgggggaggc ttagtgcagc ctggagggtc ccggaaactc 60
<210> 3
   <211> 108
   <212> PRT
   <213> Artificial Sequence
<220>
   <223> Light chain variable region sequence of 2161D2 clone antibody (Protein)
<400> 3
   Asp Ile Val Leu Thr Gln Thr Asn Ala Ser Leu Ala Val Ser Leu Gly
   1               5                   10                  15
   Gln Arg Ala Thr Ile Ser Tyr Arg Ala Ser Lys Ser Val Ser Thr Ser
               20                  25                  30
   Gly Tyr Ser Tyr Met His Trp Asn Gln Gln Lys Pro Gly Gln Pro Pro
           35                  40                  45
   Arg Leu Leu Ile Tyr Leu Val Ser Asn Leu Glu Ser Gly Val Pro Val
       50                  55                  60
   Arg Phe Ser Gly Ser Gly Ser Gly Thr Asp Phe Thr Leu Asn Ile His
   65                  70                  75                  80
   Pro Val Glu Glu Glu Asp Ala Ala Thr Tyr Tyr Cys Gln His Ile Arg
                   85                  90                  95
   Glu Leu Thr Arg Ser Glu Gly Gly Pro Ser Trp Lys
               100                 105
<210> 4
   <211> 356
   <212> DNA
   <213> Artificial Sequence
<220>
   <223> Light chain variable region sequence of 2161D2 clone antibody (DNA)
<400> 4
   gatattgtgc tcacccaaac taacgcttcc ttagctgtat ctctggggca gagggccacc 60
<210> 5
   <211> 112
   <212> PRT
   <213> Artificial Sequence
<220>
   <223> Heavy chain variable region sequence of 2164H5 clone antibody (Protein)
<400> 5
   Glu Val Gln Leu Gln Glu Ser Gly Gly Gly Leu Val Gln Pro Gly Gly
   1               5                   10                  15
   Ser Arg Lys Leu Ser Cys Ala Ala Ser Gly Phe Thr Phe Ser Ser Phe
               20                  25                  30
   Gly Met His Trp Val Arg Gln Ala Pro Glu Lys Gly Leu Glu Trp Val
           35                  40                  45
   Ala Tyr Ile Ser Ser Gly Ser Ser Thr Leu His Tyr Ala Asp Thr Val
       50                  55                  60
   Lys Gly Arg Phe Thr Ile Ser Arg Asp Asn Pro Lys Asn Thr Leu Phe
   65                  70                  75                  80
   Leu Gln Met Lys Leu Pro Ser Leu Cys Tyr Gly Leu Leu Gly Ser Arg
                   85                  90                  95
   Asn Leu Ser His Arg Leu Leu Ser Gln Asn Asp Thr Pro Ile Cys Leu
               100                 105                 110
<210> 6
   <211> 337
   <212> DNA
   <213> Artificial Sequence
<220>
   <223> Heavy chain variable region sequence of 2164H5 clone antibody (DNA)
<400> 6
   gaagttcagc tgcaggagtc tgggggaggc ttagtgcagc ctggagggtc ccggaaactc 60
   tcctgtgcag cctctggatt cactttcagt agctttggaa tgcactgggt tcgtcaggct 120
   ccagagaagg ggctggagtg ggtcgcatac attagtagtg gcagtagtac cctccactat 180
   gcagacacag tgaagggccg attcaccatc tccagagaca atcccaagaa caccctgttc 240
   ctgcaaatga aactaccctc actatgctat ggactactgg ggtcaaggaa cctcagtcac 300
   cgtctcctca gccaaaacga cacccccatc tgtctat 337
<210> 7
   <211> 120
   <212> PRT
   <213> Artificial Sequence
<220>
   <223> Light chain variable region sequence of 2164H5 clone antibody (Protein)
<400> 7
   Asp Ile Val Ile Thr Gln Ser Asn Ala Ser Leu Ala Val Ser Leu Gly
   1               5                   10                  15
   Gln Arg Ala Thr Ile Ser Ser Arg Ala Ser Glu Ser Leu Glu Tyr Tyr
               20                  25                  30
   Gly Ile Ser Leu Met His Trp Tyr Leu Gln Lys Pro Gly Gln Pro Pro
           35                  40                  45
   Lys Leu Leu Ile Tyr Ala Ala Ser Asn Val Glu Ser Gly Val Pro Ala
       50                  55                  60
   Arg Phe Ser Gly Ser Gly Ser Gly Thr Asp Phe Ser Leu Asn Ile His
   65                  70                  75                  80
   Pro Val Glu Glu Asp Asp Ile Ala Met Tyr Phe Cys Gln Gln Ser Arg
                   85                  90                  95
   Lys Val Pro Ser Thr Phe Gly Gly Gly Thr Lys Leu Glu Ile Lys Arg
               100                 105                 110
   Ala Asp Ala Ala Pro Thr Val Ser
           115                 120
<210> 8
   <211> 360
   <212> DNA
   <213> Artificial Sequence
<220>
   <223> Light chain variable region sequence of 2164H5 clone antibody (DNA)
<400> 8
   gatattgtga tcacccagtc taacgcctct ttggctgtgt ctctggggca gagagccacc        60
   atctccagca gagccagtga aagtcttgaa tattatggca taagtttaat gcactggtac       120
   ctacagaaac caggacagcc acccaaactc ctcatctatg ctgcatccaa cgtagaatct       180
   ggggtccctg ccaggtttag tggcagtggg tcagggacag acttcagcct caacatccat       240
   cctgtggagg aggatgatat tgcaatgtat ttctgtcagc aaagtaggaa ggttccttcg       300
   acgttcggtg gaggcaccaa gctggaaatc aaacgggctg atgctgcacc aactgtatcc       360
<210> 9
   <211> 125
   <212> PRT
   <213> Artificial Sequence
<220>
   <223> Heavy chain variable region sequence of 2172B12 clone antibody (Protein)
<400> 9
   Glu Val Gln Leu Glu Gln Ser Gly Gly Gly Leu Val Lys Pro Gly Gly
   1               5                   10                  15
   Ser Leu Lys Leu Ser Cys Ala Ala Ser Gly Ile Thr Phe Ser Gly Tyr
               20                  25                  30
   Ala Met Ser Trp Ile Arg Gln Thr Pro Glu Lys Arg Leu Glu Trp Val
           35                  40                  45
   Ala Thr Ile Ser Ser Gly Gly Asn Tyr Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Val Ser Ser Leu Arg Ser Glu Asp Thr Ala Ile Tyr Tyr Cys
                   85                  90                  95
   Ala Arg Leu Tyr Tyr Gly Phe Asp Tyr Trp Gly Gln Gly Thr Thr Leu
               100                 105                 110
   Thr Val Ser Ser Ala Lys Thr Thr Pro Pro Ser Val Tyr
           115                 120                 125
<210> 10
   <211> 375
   <212> DNA
   <213> Artificial Sequence
<220>
   <223> Heavy chain variable region sequence of 2172B12 clone antibody **(DNA)**
<400> 10
   gaagtccagc tggagcagtc agggggaggc cttgtgaagc ctggagggtc cctaaaactc      60
   tcctgtgcag cctctggaat tactttcagt ggctatgcca tgtcttggat tcgccagact     120
   ccggagaaga gactggagtg ggtcgcaacc attagtagtg gtggtaatta cacctactat     180
   ccagacactg tgaaggggcg tttcaccatc tccagagaca atgccaaaaa caccctatac     240
   ttacaagtga gcagtctgag gtctgaggac acggccatat attactgtgc aagactttac     300
   tacggattcg actactgggg ccaaggcacc actctcacag tctcctcagc caaaacgaca     360
   cccccatctg tctat                                                      375
<210> 11
   <211> 117
   <212> PRT
   <213> Artificial Sequence
<220>
   <223> Light chain variable region sequence of 2172B12 clone antibody (Protein)
<400> 11
   Asp Ile Val Leu Thr Gln Ser Asn Lys Ile Met Ser Ala Ser Val Gly
   1               5                   10                  15
   Asp Arg Val Ser Val Thr Cys Lys Ala Ser Gln Asn Val Asp Ser Asn
               20                  25                  30
   Val Ala Trp Tyr Gln Gln Lys Pro Gly His Ser Pro Lys Ala Leu Ile
           35                  40                  45
   Tyr Ser Ala Ser Tyr Arg Tyr Ser Arg Val Pro Asp Arg Phe Thr Gly
       50                  55                  60
   Ser Gly Ser Gly Thr Asp Phe Thr Leu Thr Ile Thr Asn Val Gln Ser
   65                  70                  75                  80
   Glu Asp Leu Ala Asp Tyr Phe Cys Gln Gln Tyr His Ser Tyr Pro Leu
                   85                  90                  95
   Leu Thr Phe Gly Ala Gly Thr Lys Leu Glu Leu Lys Arg Ala Asp Ala
               100                 105                 110
   Ala Pro Thr Val Ser
           115
<210> 12
   <211> 351
   <212> DNA
   <213> Artificial Sequence
<220>
   <223> Light chain variable region sequence of 2172B12 clone antibody (DNA)
<400> 12
   gacattgtgc tcacccaatc taacaaaatc atgtccgcat cagtaggaga ccgggtcagt        60
   gtcacctgca aggccagtca gaatgtggat agtaatgtgg cctggtatca acagaaacct       120
   ggacattctc ccaaagcact aatttattcg gcatcctacc ggtacagtag agtccctgat       180
   cgcttcacag gcagtggatc tgggacagat ttcactctca ccatcaccaa tgtgcagtct       240
   gaagacttgg cagactattt ctgtcagcaa tatcacagct atcctcttct cacgttcggt       300
   gctgggacca agctggagct gaaacgggct gatgctgcac caactgtatc c                351
<210> 13
   <211> 128
   <212> PRT
   <213> Artificial Sequence
<220>
   <223> Heavy chain variable region sequence of 2166G4 clone antibody (Protein)
<400> 13
   Glu Val Gln Leu Glu Glu Ser Gly Ala Glu Leu Val Lys Pro Gly Ala
   1               5                   10                  15
   Ser Val Lys Leu Ser Cys Lys Ala Ser Gly Tyr Ser Phe Thr Ser Tyr
               20                  25                  30
   Tyr Met Tyr Trp Val Lys Glu Arg Pro Gly Gln Gly Leu Glu Trp Ile
           35                  40                  45
   Gly Gly Ile Asn Pro Thr Asn Gly Asn Thr Asp Phe Asn Glu Asn Phe
       50                  55                  60
   Lys Asn Lys Ala Thr Leu Thr Val Asp Lys Ser Ser Ser Thr Val Tyr
   65                  70                  75                  80
   Met Arg Leu Ser Gly Leu Thr Ser Glu Asp Ser Ala Val Tyr Tyr Cys
                   85                  90                  95
   Ser Arg Ser Lys Tyr Gly Tyr Ala Trp Phe Ala Tyr Trp Gly Arg Gly
               100                 105                 110
   Thr Leu Val Thr Val Ser Ala Ala Lys Thr Thr Pro Pro Ser Val Tyr
           115                 120                 125
<210> 14
   <211> 384
   <212> DNA
   <213> Artificial Sequence
<220>
   <223> Heavy chain variable region sequence of 2166G4 clone antibody (DNA)
<400> 14
   gaggtccagc tggaggagtc tggggctgaa ctggtgaagc ctggggcttc agtgaagttg       60
   tcctgcaagg cttctggcta ctccttcacc agctactata tgtactgggt gaaggagagg      120
   cctggacaag gccttgagtg gattgggggg atcaatccta ccaatggtaa tactgacttc      180
   aatgagaact tcaagaacaa ggccacactg actgtagaca aatcctccag cacagtctat      240
   atgcgactca gcggcctgac atctgaggac tctgcggtct attattgttc aagatctaaa      300
   tatggttacg cctggtttgc ttactggggc cgagggactc tggtcactgt ctctgcagcc      360
   aaaacgacac ccccatctgt ctat                                             384
<210> 15
   <211> 119
   <212> PRT
   <213> Artificial Sequence
<220>
   <223> Light chain variable region sequence of 2166G4 clone antibody (Protein)
<400> 15
   Asp Ile Val Leu Thr Gln Ser His Ala Ser Leu Thr Val Ser Leu Gly
   1               5                   10                  15
   Gln Arg Ala Ala Ile Ser Cys Arg Ser Ser Gln Ser Val Asn Thr Ser
               20                  25                  30
   Ala Tyr Ser Tyr Val His Ser Tyr Gln Gln Arg Pro Gly Gln Pro Pro
           35                  40                  45
   Lys Leu Leu Ile Lys Tyr Ala Ser Asn Leu Glu Ser Gly Val Pro Ala
       50                  55                  60
   Arg Phe Ser Val Ser Gly Ser Gly Thr Asp Phe Thr Leu Asn Ile His
   65                  70                  75                  80
   Ser Val Glu Glu Glu Asp Pro Thr Thr Tyr Asp Cys Gln His Ser Trp
                   85                  90                  95
   Glu Ile His Ser Arg Ser Ala Arg Gly Gln Ser Trp Lys Tyr Asn Gly
               100                 105                 110
   Leu Met Leu Gln Thr Val Ser
           115
<210> 16
   <211> 357
   <212> DNA
   <213> Artificial Sequence
<220>
   <223> Light chain variable region sequence of 2166G4 clone antibody (DNA)
<400> 16
   gacattgtgc tgacccaatc tcacgcttcc ttaactgtat ctctggggca gagggccgcc       60
   atctcatgca ggtccagcca aagtgtcaat acatctgcct atagttatgt gcactcctac      120
   caacagagac caggccagcc acccaaactc ctcatcaagt atgcatccaa cctagaatct      180
   ggggtccctg ccaggttcag tgtcagtggg tctgggacag acttcaccct caacatccat      240
   tctgtggagg aggaggatcc tacaacatat gactgtcagc acagttggga gattcattca      300
   cgttcggctc gggggcaaag ttggaaatat aacgggctga tgctgcaaac tgtatcc         357
<210> 17
   <211> 2322
   <212> PRT
   <213> Artificial Sequence
<220>
   <223> NG2/CSPG4 ectodomain
<400> 17
   Met Gln Ser Gly Pro Arg Pro Pro Leu Pro Ala Pro Gly Leu Ala Leu
   1               5                   10                  15
   Ala Leu Thr Leu Thr Met Leu Ala Arg Leu Ala Ser Ala Ala Ser Phe
               20                  25                  30
   Phe Gly Glu Asn His Leu Glu Val Pro Val Ala Thr Ala Leu Thr Asp
           35                  40                  45
   Ile Asp Leu Gln Leu Gln Phe Ser Thr Ser Gln Pro Glu Ala Leu Leu
       50                  55                  60
   Leu Leu Ala Ala Gly Pro Ala Asp His Leu Leu Leu Gln Leu Tyr Ser
   65                  70                  75                  80
   Gly Arg Leu Gln Val Arg Leu Val Leu Gly Gln Glu Glu Leu Arg Leu
                   85                  90                  95
   Gln Thr Pro Ala Glu Thr Leu Leu Ser Asp Ser Ile Pro His Thr Val
               100                 105                 110
   Val Leu Thr Val Val Glu Gly Trp Ala Thr Leu Ser Val Asp Gly Phe
           115                 120                 125
   Leu Asn Ala Ser Ser Ala Val Pro Gly Ala Pro Leu Glu Val Pro Tyr
       130                 135                 140
   Gly Leu Phe Val Gly Gly Thr Gly Thr Leu Gly Leu Pro Tyr Leu Arg
   145                 150                 155                 160
   Gly Thr Ser Arg Pro Leu Arg Gly Cys Leu His Ala Ala Thr Leu Asn
                   165                 170                 175
   Gly Arg Ser Leu Leu Arg Pro Leu Thr Pro Asp Val His Glu Gly Cys
               180                 185                 190
   Ala Glu Glu Phe Ser Ala Ser Asp Asp Val Ala Leu Gly Phe Ser Gly
           195                 200                 205
   Pro His Ser Leu Ala Ala Phe Pro Ala Trp Gly Thr Gln Asp Glu Gly
       210                 215                 220
   Thr Leu Glu Phe Thr Leu Thr Thr Gln Ser Arg Gln Ala Pro Leu Ala
   225                 230                 235                 240
   Phe Gln Ala Gly Gly Arg Arg Gly Asp Phe Ile Tyr Val Asp Ile Phe
                   245                 250                 255
   Glu Gly His Leu Arg Ala Val Val Glu Lys Gly Gln Gly Thr Val Leu
               260                 265                 270
   Leu His Asn Ser Val Pro Val Ala Asp Gly Gln Pro His Glu Val Ser
           275                 280                 285
   Val His Ile Asn Ala His Arg Leu Glu Ile Ser Val Asp Gln Tyr Pro
       290                 295                 300
   Thr His Thr Ser Asn Arg Gly Val Leu Ser Tyr Leu Glu Pro Arg Gly
   305                 310                 315                 320
   Ser Leu Leu Leu Gly Gly Leu Asp Ala Glu Ala Ser Arg His Leu Gln
                   325                 330                 335
   Glu His Arg Leu Gly Leu Thr Pro Glu Ala Thr Asn Ala Ser Leu Leu
               340                 345                 350
   Gly Cys Met Glu Asp Leu Ser Val Asn Gly Gln Arg Arg Gly Leu Arg
           355                 360                 365
   Glu Ala Leu Leu Thr Arg Asn Met Ala Ala Gly Cys Arg Leu Glu Glu
       370                 375                 380
   Glu Glu Tyr Glu Asp Asp Ala Tyr Gly His Tyr Glu Ala Phe Ser Thr
   385                 390                 395                 400
   Leu Ala Pro Glu Ala Trp Pro Ala Met Glu Leu Pro Glu Pro Cys Val
                   405                 410                 415
   Pro Glu Pro Gly Leu Pro Pro Val Phe Ala Asn Phe Thr Gln Leu Leu
               420                 425                 430
   Thr Ile Ser Pro Leu Val Val Ala Glu Gly Gly Thr Ala Trp Leu Glu
           435                 440                 445
   Trp Arg His Val Gln Pro Thr Leu Asp Leu Met Glu Ala Glu Leu Arg
       450                 455                 460
   Lys Ser Gln Val Leu Phe Ser Val Thr Arg Gly Ala Arg His Gly Glu
   465                 470                 475                 480
   Leu Glu Leu Asp Ile Pro Gly Ala Gln Ala Arg Lys Met Phe Thr Leu
                   485                 490                 495
   Leu Asp Val Val Asn Arg Lys Ala Arg Phe Ile His Asp Gly Ser Glu
               500                 505                 510
   Asp Thr Ser Asp Gln Leu Val Leu Glu Val Ser Val Thr Ala Arg Val
           515                 520                 525
   Pro Met Pro Ser Cys Leu Arg Arg Gly Gln Thr Tyr Leu Leu Pro Ile
       530                 535                 540
   Gln Val Asn Pro Val Asn Asp Pro Pro His Ile Ile Phe Pro His Gly
   545                 550                 555                 560
   Ser Leu Met Val Ile Leu Glu His Thr Gln Lys Pro Leu Gly Pro Glu
                   565                 570                 575
   Val Phe Gln Ala Tyr Asp Pro Asp Ser Ala Cys Glu Gly Leu Thr Phe
               580                 585                 590
   Gln Val Leu Gly Thr Ser Ser Gly Leu Pro Val Glu Arg Arg Asp Gln
           595                 600                 605
   Pro Gly Glu Pro Ala Thr Glu Phe Ser Cys Arg Glu Leu Glu Ala Gly
       610                 615                 620
   Ser Leu Val Tyr Val His Arg Gly Gly Pro Ala Gln Asp Leu Thr Phe
   625                 630                 635                 640
   Arg Val Ser Asp Gly Leu Gln Ala Ser Pro Pro Ala Thr Leu Lys Val
                   645                 650                 655
   Val Ala Ile Arg Pro Ala Ile Gln Ile His Arg Ser Thr Gly Leu Arg
               660                 665                 670
   Leu Ala Gln Gly Ser Ala Met Pro Ile Leu Pro Ala Asn Leu Ser Val
           675                 680                 685
   Glu Thr Asn Ala Val Gly Gln Asp Val Ser Val Leu Phe Arg Val Thr
       690                 695                 700
   Gly Ala Leu Gln Phe Gly Glu Leu Gln Lys Gln Gly Ala Gly Gly Val
   705                 710                 715                 720
   Glu Gly Ala Glu Trp Trp Ala Thr Gln Ala Phe His Gln Arg Asp Val
                   725                 730                 735
   Glu Gln Gly Arg Val Arg Tyr Leu Ser Thr Asp Pro Gln His His Ala
               740                 745                 750
   Tyr Asp Thr Val Glu Asn Leu Ala Leu Glu Val Gln Val Gly Gln Glu
           755                 760                 765
   Ile Leu Ser Asn Leu Ser Phe Pro Val Thr Ile Gln Arg Ala Thr Val
       770                 775                 780
   Trp Met Leu Arg Leu Glu Pro Leu His Thr Gln Asn Thr Gln Gln Glu
   785                 790                 795                 800
   Thr Leu Thr Thr Ala His Leu Glu Ala Thr Leu Glu Glu Ala Gly Pro
                   805                 810                 815
   Ser Pro Pro Thr Phe His Tyr Glu Val Val Gln Ala Pro Arg Lys Gly
               820                 825                 830
   Asn Leu Gln Leu Gln Gly Thr Arg Leu Ser Asp Gly Gln Gly Phe Thr
           835                 840                 845
   Gln Asp Asp Ile Gln Ala Gly Arg Val Thr Tyr Gly Ala Thr Ala Arg
   850 855 860
   Ala Ser Glu Ala Val Glu Asp Thr Phe Arg Phe Arg Val Thr Ala Pro
   865 870 875 880
   Pro Tyr Phe Ser Pro Leu Tyr Thr Phe Pro Ile His Ile Gly Gly Asp
   885 890 895
   Pro Asp Ala Pro Val Leu Thr Asn Val Leu Leu Val Val Pro Glu Gly
   900 905 910
   Gly Glu Gly Val Leu Ser Ala Asp His Leu Phe Val Lys Ser Leu Asn
   915 920 925
   Ser Ala Ser Tyr Leu Tyr Glu Val Met Glu Arg Pro Arg His Gly Arg
   930 935 940
   Leu Ala Trp Arg Gly Thr Gln Asp Lys Thr Thr Met Val Thr Ser Phe
   945 950 955 960
   Thr Asn Glu Asp Leu Leu Arg Gly Arg Leu Val Tyr Gln His Asp Asp
   965 970 975
   Ser Glu Thr Thr Glu Asp Asp Ile Pro Phe Val Ala Thr Arg Gln Gly
   980 985 990
   Glu Ser Ser Gly Asp Met Ala Trp Glu Glu Val Arg Gly Val Phe Arg
   995 1000 1005
   Val Ala Ile Gln Pro Val Asn Asp His Ala Pro Val Gln Thr Ile
   1010 1015 1020
   Ser Arg Ile Phe His Val Ala Arg Gly Gly Arg Arg Leu Leu Thr
   1025 1030 1035
   Thr Asp Asp Val Ala Phe Ser Asp Ala Asp Ser Gly Phe Ala Asp
   1040 1045 1050
   Ala Gln Leu Val Leu Thr Arg Lys Asp Leu Leu Phe Gly Ser Ile
   1055 1060 1065
   Val Ala Val Asp Glu Pro Thr Arg Pro Ile Tyr Arg Phe Thr Gln
   1070 1075 1080
   Glu Asp Leu Arg Lys Arg Arg Val Leu Phe Val His Ser Gly Ala
   1085 1090 1095
   Asp Arg  Gly Trp Ile Gln Leu  Gln Val Ser Asp Gly  Gln His Gln
       1100                 1105                 1110
   Ala Thr  Ala Leu Leu Glu Val  Gln Ala Ser Glu Pro  Tyr Leu Arg
       1115                 1120                 1125
   Val Ala  Asn Gly Ser Ser Leu  Val Val Pro Gln Gly  Gly Gln Gly
       1130                 1135                 1140
   Thr Ile  Asp Thr Ala Val Leu  His Leu Asp Thr Asn  Leu Asp Ile
       1145                 1150                 1155
   Arg Ser  Gly Asp Glu Val His  Tyr His Val Thr Ala  Gly Pro Arg
       1160                 1165                 1170
   Trp Gly  Gln Leu Val Arg Ala  Gly Gln Pro Ala Thr  Ala Phe Ser
       1175                 1180                 1185
   Gln Gln  Asp Leu Leu Asp Gly  Ala Val Leu Tyr Ser  His Asn Gly
       1190                 1195                 1200
   Ser Leu  Ser Pro Arg Asp Thr  Met Ala Phe Ser Val  Glu Ala Gly
       1205                 1210                 1215
   Pro Val  His Thr Asp Ala Thr  Leu Gln Val Thr Ile  Ala Leu Glu
       1220                 1225                 1230
   Gly Pro  Leu Ala Pro Leu Lys  Leu Val Arg His Lys  Lys Ile Tyr
       1235                 1240                 1245
   Val Phe  Gln Gly Glu Ala Ala  Glu Ile Arg Arg Asp  Gln Leu Glu
       1250                 1255                 1260
   Ala Ala  Gln Glu Ala Val Pro  Pro Ala Asp Ile Val  Phe Ser Val
       1265                 1270                 1275
   Lys Ser  Pro Pro Ser Ala Gly  Tyr Leu Val Met Val  Ser Arg Gly
       1280                 1285                 1290
   Ala Leu  Ala Asp Glu Pro Pro  Ser Leu Asp Pro Val  Gln Ser Phe
       1295                 1300                 1305
   Ser Gln  Glu Ala Val Asp Thr  Gly Arg Val Leu Tyr  Leu His Ser
       1310                 1315                 1320
   Arg Pro  Glu Ala Trp Ser Asp  Ala Phe Ser Leu Asp  Val Ala Ser
       1325                 1330                 1335
   Gly Leu  Gly Ala Pro Leu Glu  Gly Val Leu Val Glu  Leu Glu Val
       1340                 1345                 1350
   Leu Pro  Ala Ala Ile Pro Leu  Glu Ala Gln Asn Phe  Ser Val Pro
       1355                 1360                 1365
   Glu Gly  Gly Ser Leu Thr Leu  Ala Pro Pro Leu Leu  Arg Val Ser
       1370                 1375                 1380
   Gly Pro  Tyr Phe Pro Thr Leu  Leu Gly Leu Ser Leu  Gln Val Leu
       1385                 1390                 1395
   Glu Pro  Pro Gln His Gly Ala  Leu Gln Lys Glu Asp  Gly Pro Gln
       1400                 1405                 1410
   Ala Arg  Thr Leu Ser Ala Phe  Ser Trp Arg Met Val  Glu Glu Gln
       1415                 1420                 1425
   Leu Ile  Arg Tyr Val His Asp  Gly Ser Glu Thr Leu  Thr Asp Ser
       1430                 1435                 1440
   Phe Val  Leu Met Ala Asn Ala  Ser Glu Met Asp Arg  Gln Ser His
       1445                 1450                 1455
   Pro Val  Ala Phe Thr Val Thr  Val Leu Pro Val Asn  Asp Gln Pro
       1460                 1465                 1470
   Pro Ile  Leu Thr Thr Asn Thr  Gly Leu Gln Met Trp  Glu Gly Ala
       1475                 1480                 1485
   Thr Ala  Pro Ile Pro Ala Glu  Ala Leu Arg Ser Thr  Asp Gly Asp
       1490                 1495                 1500
   Ser Gly  Ser Glu Asp Leu Val  Tyr Thr Ile Glu Gln  Pro Ser Asn
       1505                 1510                 1515
   Gly Arg  Val Val Leu Arg Gly  Ala Pro Gly Thr Glu  Val Arg Ser
       1520                 1525                 1530
   Phe Thr  Gln Ala Gln Leu Asp  Gly Gly Leu Val Leu  Phe Ser His
       1535                 1540                 1545
   Arg Gly  Thr Leu Asp Gly Gly  Phe Arg Phe Arg Leu  Ser Asp Gly
       1550                 1555                 1560
   Glu His  Thr Ser Pro Gly His  Phe Phe Arg Val Thr  Ala Gln Lys
       1565                 1570                 1575
   Gln Val Leu Leu Ser Leu Lys Gly Ser Gln Thr Leu Thr Val Cys
   1580 1585 1590
   Pro Gly Ser Val Gln Pro Leu Ser Ser Gln Thr Leu Arg Ala Ser
   1595 1600 1605
   Ser Ser Ala Gly Thr Asp Pro Gln Leu Leu Leu Tyr Arg Val Val
   1610 1615 1620
   Arg Gly Pro Gln Leu Gly Arg Leu Phe His Ala Gln Gln Asp Ser
   1625 1630 1635
   Thr Gly Glu Ala Leu Val Asn Phe Thr Gln Ala Glu Val Tyr Ala
   1640 1645 1650
   Gly Asn Ile Leu Tyr Glu His Glu Met Pro Pro Glu Pro Phe Trp
   1655 1660 1665
   Glu Ala His Asp Thr Leu Glu Leu Gln Leu Ser Ser Pro Pro Ala
   1670 1675 1680
   Arg Asp Val Ala Ala Thr Leu Ala Val Ala Val Ser Phe Glu Ala
   1685 1690 1695
   Ala Cys Pro Gln Arg Pro Ser His Leu Trp Lys Asn Lys Gly Leu
   1700 1705 1710
   Trp Val Pro Glu Gly Gln Arg Ala Arg Ile Thr Val Ala Ala Leu
   1715 1720 1725
   Asp Ala Ser Asn Leu Leu Ala Ser Val Pro Ser Pro Gln Arg Ser
   1730 1735 1740
   Glu His Asp Val Leu Phe Gln Val Thr Gln Phe Pro Ser Arg Gly
   1745 1750 1755
   Gln Leu Leu Val Ser Glu Glu Pro Leu His Ala Gly Gln Pro His
   1760 1765 1770
   Phe Leu Gln Ser Gln Leu Ala Ala Gly Gln Leu Val Tyr Ala His
   1775 1780 1785
   Gly Gly Gly Gly Thr Gln Gln Asp Gly Phe His Phe Arg Ala His
   1790 1795 1800
   Leu Gln Gly Pro Ala Gly Ala Ser Val Ala Gly Pro Gln Thr Ser
   1805 1810 1815
   Glu Ala Phe Ala Ile Thr Val Arg Asp Val Asn Glu Arg Pro Pro
   1820 1825 1830
   Gln Pro Gln Ala Ser Val Pro Leu Arg Leu Thr Arg Gly Ser Arg
   1835 1840 1845
   Ala Pro Ile Ser Arg Ala Gln Leu Ser Val Val Asp Pro Asp Ser
   1850 1855 1860
   Ala Pro Gly Glu Ile Glu Tyr Glu Val Gln Arg Ala Pro His Asn
   1865 1870 1875
   Gly Phe Leu Ser Leu Val Gly Gly Gly Leu Gly Pro Val Thr Arg
   1880 1885 1890
   Phe Thr Gln Ala Asp Val Asp Ser Gly Arg Leu Ala Phe Val Ala
   1895 1900 1905
   Asn Gly Ser Ser Val Ala Gly Ile Phe Gln Leu Ser Met Ser Asp
   1910 1915 1920
   Gly Ala Ser Pro Pro Leu Pro Met Ser Leu Ala Val Asp Ile Leu
   1925 1930 1935
   Pro Ser Ala Ile Glu Val Gln Leu Arg Ala Pro Leu Glu Val Pro
   1940 1945 1950
   Gln Ala Leu Gly Arg Ser Ser Leu Ser Gln Gln Gln Leu Arg Val
   1955 1960 1965
   Val Ser Asp Arg Glu Glu Pro Glu Ala Ala Tyr Arg Leu Ile Gln
   1970 1975 1980
   Gly Pro Gln Tyr Gly His Leu Leu Val Gly Gly Arg Pro Thr Ser
   1985 1990 1995
   Ala Phe Ser Gln Phe Gln Ile Asp Gln Gly Glu Val Val Phe Ala
   2000 2005 2010
   Phe Thr Asn Phe Ser Ser Ser His Asp His Phe Arg Val Leu Ala
   2015 2020 2025
   Leu Ala Arg Gly Val Asn Ala Ser Ala Val Val Asn Val Thr Val
   2030 2035 2040
   Arg Ala  Leu Leu His Val Trp  Ala Gly Gly Pro Trp  Pro Gln Gly
       2045                 2050                 2055
   Ala Thr  Leu Arg Leu Asp Pro  Thr Val Leu Asp Ala  Gly Glu Leu
       2060                 2065                 2070
   Ala Asn  Arg Thr Gly Ser Val  Pro Arg Phe Arg Leu  Leu Glu Gly
       2075                 2080                 2085
   Pro Arg  His Gly Arg Val Val  Arg Val Pro Arg Ala  Arg Thr Glu
       2090                 2095                 2100
   Pro Gly  Gly Ser Gln Leu Val  Glu Gln Phe Thr Gln  Gln Asp Leu
       2105                 2110                 2115
   Glu Asp  Gly Arg Leu Gly Leu  Glu Val Gly Arg Pro  Glu Gly Arg
       2120                 2125                 2130
   Ala Pro  Gly Pro Ala Gly Asp  Ser Leu Thr Leu Glu  Leu Trp Ala
       2135                 2140                 2145
   Gln Gly  Val Pro Pro Ala Val  Ala Ser Leu Asp Phe  Ala Thr Glu
       2150                 2155                 2160
   Pro Tyr  Asn Ala Ala Arg Pro  Tyr Ser Val Ala Leu  Leu Ser Val
       2165                 2170                 2175
   Pro Glu  Ala Ala Arg Thr Glu  Ala Gly Lys Pro Glu  Ser Ser Thr
       2180                 2185                 2190
   Pro Thr  Gly Glu Pro Gly Pro  Met Ala Ser Ser Pro  Glu Pro Ala
       2195                 2200                 2205
   Val Ala  Lys Gly Gly Phe Leu  Ser Phe Leu Glu Ala  Asn Met Phe
       2210                 2215                 2220
   Ser Val  Ile Ile Pro Met Cys  Leu Val Leu Leu Leu  Leu Ala Leu
       2225                 2230                 2235
   Ile Leu  Pro Leu Leu Phe Tyr  Leu Arg Lys Arg Asn  Lys Thr Gly
       2240                 2245                 2250
   Lys His  Asp Val Gln Val Leu  Thr Ala Lys Pro Arg  Asn Gly Leu
       2255                 2260                 2265
   Ala Gly  Asp Thr Glu Thr Phe  Arg Lys Val Glu Pro  Gly Gln Ala
       2270                 2275                 2280
   Ile Pro  Leu Thr Ala Val Pro  Gly Gln Gly Pro Pro  Pro Gly Gly
       2285                 2290                 2295
   Gln Pro  Asp Pro Glu Leu Leu  Gln Phe Cys Arg Thr  Pro Asn Pro
       2300                 2305                 2310
   Ala Leu  Lys Asn Gly Gln Tyr  Trp Val
       2315                 2320
<210> 18
   <211> 24
   <212> DNA
   <213> Artificial Sequence
<220>
   <223> CDR1 Heavy chain variable region 2161D2
<400> 18
   ggattcactt tcagtagctt tgga 24
<210> 19
   <211> 24
   <212> DNA
   <213> Artificial Sequence
<220>
   <223> CDR2 Heavy chain variable region 2161D2
<400> 19
   attagtagtg gcagtagtac cctc 24
<210> 20
   <211> 30
   <212> DNA
   <213> Artificial Sequence
<220>
   <223> CDR1 Light chain variable region 2161D2
<400> 20
   aaaagtgtca gtacatctgg ctatagttat 30
<210> 21
   <211> 9
   <212> DNA
   <213> Artificial Sequence
<220>
   <223> CDR2 Light chain variable region 2161D2
<400> 21
   cttgtatcc 9
<210> 22
   <211> 24
   <212> DNA
   <213> Artificial Sequence
<220>
   <223> CDR3 Light chain variable region 2161D2
<400> 22
   cagcacatta gggagcttac acgt 24
<210> 23
   <211> 24
   <212> DNA
   <213> Artificial Sequence
<220>
   <223> CDR1 Heavy chain variable region 2164H5
<400> 23
   ggattcactt tcagtagctt tgga 24
<210> 24
   <211> 24
   <212> DNA
   <213> Artificial Sequence
<220>
   <223> CDR2 Heavy chain variable region 2164H5
<400> 24
   attagtagtg gcagtagtac cctc 24
<210> 25
   <211> 30
   <212> DNA
   <213> Artificial Sequence
<220>
   <223> CDR1 Light chain variable region 2164H5
<400> 25
   gaaagtcttg aatattatgg cataagttta 30
<210> 26
   <211> 9
   <212> DNA
   <213> Artificial Sequence
<220>
   <223> CDR2 Light chain variable region 2164H5
<400> 26
   gctgcatcc 9
<210> 27
   <211> 27
   <212> DNA
   <213> Artificial Sequence
<220>
   <223> CDR3 Light chain variable region 2164H5
<400> 27
   cagcaaagta ggaaggttcc ttcgacg 27
<210> 28
   <211> 24
   <212> DNA
   <213> Artificial Sequence
<220>
   <223> CDR1 Heavy chain variable region 2166G4
<400> 28
   ggctactcct tcaccagcta ctat 24
<210> 29
   <211> 24
   <212> DNA
   <213> Artificial Sequence
<220>
   <223> CDR2 Heavy chain variable region 2166G4
<400> 29
   atcaatccta ccaatggtaa tact 24
<210> 30
   <211> 27
   <212> DNA
   <213> Artificial Sequence
<220>
   <223> CDR3 Heavy chain variable region 2166G4
<400> 30
   tcaagatcta aatatggtta cgcctgg 27
<210> 31
   <211> 30
   <212> DNA
   <213> Artificial Sequence
<220>
   <223> CDR1 Light chain variable region 2166G4
<400> 31
   caaagtgtca atacatctgc ctatagttat 30
<210> 32
   <211> 9
   <212> DNA
   <213> Artificial Sequence
<220>
   <223> CDR2 Light chain variable region 2166G4
<400> 32
   tatgcatcc 9
<210> 33
   <211> 27
   <212> DNA
   <213> Artificial Sequence
<220>
   <223> CDR3 Light chain variable region 2166G4
<400> 33
   cagcacagtt gggagattca ttcacgt 27
<210> 34
   <211> 24
   <212> DNA
   <213> Artificial Sequence
<220>
   <223> CDR1 Heavy chain variable region 2172B12
<400> 34
   ggaattactt tcagtggcta tgcc 24
<210> 35
   <211> 24
   <212> DNA
   <213> Artificial Sequence
<220>
   <223> CDR2 Heavy chain variable region 2172B12
<400> 35
   attagtagtg gtggtaatta cacc 24
<210> 36
   <211> 27
   <212> DNA
   <213> Artificial Sequence
<220>
   <223> CDR3 Heavy chain variable region 2172B12
<400> 36
   gcaagacttt actacggatt cgactac 27
<210> 37
   <211> 18
   <212> DNA
   <213> Artificial Sequence
<220>
   <223> CDR1 Light chain variable region 2172B12
<400> 37
   cagaatgtgg atagtaat 18
<210> 38
   <211> 9
   <212> DNA
   <213> Artificial Sequence
<220>
   <223> CDR2 Light chain variable region 2172B12
<400> 38
   tcggcatcc 9
<210> 39
   <211> 30
   <212> DNA
   <213> Artificial Sequence
<220>
   <223> CDR3 Light chain variable region 2172B12
<400> 39
   cagcaatatc acagctatcc tcttctcacg 30

## Description

The present invention relates to an antibody capable of binding with high-affinity and high selectivity to the ectodomain of the transmembrane proteoglycan (PG) NG2/CSPG4. The invention specifically refers to said antibody possessing the ability to uniquely induce programmed cell death through canonical caspase-dependent apoptosis or authophagy, in cancer cells specifically expressing such protein, and without the participation of other exogenously added factors. Moreover, the present invention refers to a composition comprising the antibody of the invention as pharmaceutical excipient.

A further aspect of the present invention refers to the anti-NG2/CSPG4 molecule for the treatment of apoptosis -dependent pathologies, including but not restricted to cancer and related diseases.

### STATE OF THE ART

Nerve/Glia Antigen 2 (NG2) is a transmembrane proteoglycan (PG) governing the interaction of cancer cells with their microenvironment. Moreover, NG2 promotes the myriad of cellular events prompting tumor growth and cancer cell spreading. NG2 is also known as High Molecular Weight Melanoma-Associated Antigen (HMW-MAA), or simply Melanoma Cell Surface Proteoglycan (MCSP/MCSPG) because it was originally identified also on cells derived from cutaneous melanoma lesions. The human NG2 orthologous gene is coded Chondroitin Sulfate ProteoGlycan-4 (CSPG4) and it is located on chromosome 15:24q2.

NG2/CSPG4 interacts with several molecules, such as growth factors, signaling molecules, metalloproteinases and a various ECM components through its extended extracellular domain, which may associate with and control intracellularly the cell surface receptors for these ligands. Through these interactions, the PG acts a primary promoter of cell survival and apoptotic resistance, a function that is underpinned by its ability to activate the PI-3K-Akt-mTOR signaling pathways upon PCKα-induced phosphorylation of one of its cytoplasmic threonine residues (see below). In fact, cancer cells exhibiting high surface expression show enhanced chemoresistance. Intriguingly, in certain cultivated cellular models, forced overexpression of NG2/CSPG4, possibly along with downregulation of α4 integrins, may enhance anoikis and this effect is curtailed by MMP13-operated shedding of the NG2/CSPG4 ectodomain. However, evidence for such a mechanism on cells constitutively expressing high levels of NG2/CSPG4 has not been provided, nor documented to be operating *in vivo.*

In highly motile cells, such as cancer cells, NG2/CSPG4 accumulates in the advancing cytoplasmic front, preferentially in filopodial extensions and at cell-substrate contact areas. The cytoplasmic tail of NG2/CSPG4 contains two threonine residues undergoing differential phosphorylation by PKCα (Thr2256) and ERK(s) (Thr2314) depending upon the cellular events in which the PG may participate.

NG2/CSPG4 has not been identified as a factor directly promoting tumor formation; however, it has been shown to become *de novo* expressed in a number of solid and hematological neoplastic conditions, as well as to be linked to cancer-associated epithelial-mesenchymal transitions. The most striking examples of this latter transitional linkage are the appearance of NG2/CSPG4 on melanoma cells, breast, head and neck carcinomas and mesotheliomas.

Even though high transcriptional levels of the CSPG4 gene may be detected, appearance of PG on the surface of cancer cells may not always occur (i.e. the PG is primary detected intracellularly) and is likely to be a mere secondary event of tumor formation.

At present, augmented transcriptional and/or translational levels of NG2/CSPG4 have currently been disclosed in more than 30 solid tumor types (and their subvariants), and, in several of them, a certain diagnostic and/or prognostic connotation of the PG has been proposed.

This would suggest that the PG drives metastasis formation, as proposed by some experimental data in mouse models.

Furthermore, although cells engineered to overexpress NG2/CSPG4 are generally more malignant than their counterpart NG2/CSPG4-deficient cells, unequivocal experimental proof of a direct metastasis-promoting effect of the PG is currently missing.

Given its documented diagnostic impact, it appears perplexing that NG2/CSPG4 has remained largely unutilized as a biomarker for the routine clinical monitoring of cancer patients. This may be due to the failure of most of the published studies to demonstrate the independence of dysregulated NG2/CSPG4 expression from established clinical parameters.

Several *in vitro* assays support the concept that NG2/CSPG4 may affect drug sensitivity of cancer cells by reinforcing their interactions with the host microenvironment, especially with the stromal ECM, by modulating the activity of certain integrins and, through these actions, by activating pro-survival signaling cascades.

Antibodies against CSPG4 with biological activity are known from WO2010033866.

### SUMMARY OF THE INVENTION

The present invention relates to an antibody, preferably a monoclonal antibody, recognizing and binding to an antigen consisting of the ectodomain of the NG2/CSPG4 transmembrane proteoglycan according to claim 1. Preferred embodiments of the present invention are defined in dependent claims 2-7.

The antibody of the invention can be used for cell growth inhibition by exposing a cell expressing NG2/CSPG4 to a therapeutic amount of an antibody capable of binding to NG2/CSPG4.

According to an aspect of the present invention, the antibody is embodied in a bispecific antibody construct (e.g. an antibody binding both NG2/CSPG4 and another cell surface molecule and/or antigen) or a fusion protein comprised of a therapeutic amount of the antibody and another therapeutic molecule, which is preferably a cytokine or an
aptoptosis-inducing agent (e.g TRAIL or Fas Ligand), or a CAR T (Chimeric Antigen Receptor T cell) construct in which an scFv of the antibody of the invention is expressed on the surface of a CAR T cell, such as to simultaneously engage NG2/CSPG4 expressing cancer cells and endogenous T cells and favor the maintenance of the CAR T cell in proximity to the cancer cell to trigger the activation of
the engaged T cell.

Further described in the present application is the use of the antibody and the composition of the invention for selective triggering of apoptosis in cells specifically expressing NG2/CSPG4 recognized by the antibody.

A further aspect of present invention refers to the antibody and the composition of the invention for use in the treatment of an apoptosis-dependent disease, preferably cancer, more preferably solid or hematological cancers, as well as cancer-related diseases.

The present specification further describes nucleic acid sequences or amino acid sequences encoding the heavy and light chain immunoglobulins or the complementarity determining regions of the antibody.

Also described herein, but not part of the invention, is a host cell producing the antibody and its variants.

### DESCRIPTION OF THE DRAWINGS

Figure 1 shows the identification of NG2/CSPG4 isoforms and the proteolytic fragments in cells (1-A - A375 and HT1080^{NG2+} cells) and in tissues (1-B pericyte sprouts of fetal brain neovessels and in glioblastoma multiforme (GMB) lesions. Recombinant NG2/CSPG4 (NG2^{rec}) is included as a control.
Figure 2 shows the effects on cell migration of the antibodies of the invention (hybridoma clones 2172B12/C10, 2166G4/C10/D1, 2164H5/E11, 2161D2/C2 and their subclonal derivations). The cell migration model used is the wound-healing scratch assay performed using HT1080^{NG2+} as model cell line.
Figure 3 (I-II) shows the immunostaining of HT1080^{NG2+}, A375 andHS578T cancer cells and primary human brain vascular pericytes (HBVP) by using the antibodies of the invention (hybridoma clones 2172B12/C10, 2166G4/C10/D1, 2164H5/E11,2161D2/C2 and their subclonal derivations).
Figures 4 shows the effect of the antibodies of the invention (hybridoma clones 2164H5/E11, 2161D2/C2 and their subclonal derivations) on cultured tumor cells (A375, HT1080^{NG2+} and HS578) in monolayered (2D-A) or as multicellular spheroidal configurations (3D-B,C).
Figure 5 shows advanced apoptosis, measured by using the TUNEL assay, in A375 cells (A) and HT1080^{NG2+} cells (B) and induced by administration of the antibodies of the invention (hybrdioma clones 2172B12/C10, 2166G4/C10/D1, 2164H5/E11 and 2161 D2/C2 and their subclone variants) to the cells.
Figure 6 shows apoptosis measured by multiparametric assay induced by administering the antibodies of the invention (hybridoma clones 2172B12/C10, 2166G4/C10/D1, 2164H5/E11, 2161D2/C2 and their subclonal derivations) on A375, HT1080^{NG2+} cell lines.
Figure 7 shows the PARP cleavage detection by using flow cytometry on the following cancer cell lines: A375 (A), HT1080^{NG2+}(B) and HS578 (C) after administering the antibodies of the invention (hybridoma clones 2172B12/C10, 2166G4/C10/D1, 2164H5/E11, 2161D2/C2 and their subclonal derivations).
Figure 8 shows activation of caspases responsible for propagation of the extrinsic apoptotic pathway after administration of the antibodies of the invention (hybridoma clones 2172B12/C10, 2166G4/C10/D1, 2164H5/E11, 2161D2/C2 and their subclonal derivations) in 2D and 3D culture of the model cancer cell lines (A375, HT1080^{NG2+} andHT578T).
Figure 9 shows autophagy detection in the A375 cancer cell line transiently transfected with a LC3-GFP fusion construct and treated with antibodies of the invention by using the LC3-aggregation method. Figure 9 is not part of the invention.

### DETAILED DESCRIPTION OF THE INVENTION

In the context of the present invention, the term "apoptosis" means the process of programmed cell death (PCD) that may occur in multicellular organisms. Biochemical events lead to characteristic cell changes (morphology) and death. These changes include for example, blebbing, cell shrinkage, nuclear fragmentation, chromatin condensation, or chromosomal DNA fragmentation. Excessive apoptosis causes atrophy, whereas an insufficient amount results in uncontrolled cell proliferation, such as cancer.

Many pathways and signals lead to apoptosis. After a cell receives stimulus, it undergoes organized degradation of cellular organelles by activated proteolytic caspases. A cell undergoing apoptosis shows the following characteristic morphology:
- Cell shrinkage and rounding are shown because of the breakdown of the proteinaceous cytoskeleton by caspases. g
- The cytoplasm appears dense, and the organelles appear tightly packed.
- Chromatin undergoes condensation into compact patches against the nuclear envelope in a process known as pyknosis, a hallmark of apoptosis.
- The nuclear envelope becomes discontinuous and the DNA inside it is fragmented in a process referred to as karyorrhexis. The nucleus breaks into several discrete chromatin bodies or nucleosomal units due to the degradation of DNA.
- The cell membrane shows irregular buds known as blebs.
- The cell breaks apart into several vesicles called apoptotic bodies, which are then phagocytosed.

Caspases play the central role in the transduction of Death Receptor (DR) apoptotic signals.

Caspases are proteins that are highly conserved, cysteine-dependent aspartate-specific proteases. There are two types of caspases: initiator caspases and effector caspases. The activation of initiator caspases requires binding to specific oligomeric activator protein. Effector caspases are then activated by these active initiator caspases through proteolytic cleavage. The active effector caspases then proteolytically degrade a host of intracellular proteins to carry out the cell death program.

Therefore, in a preferred embodiment the apoptosis of the present invention is caspase-dependent.

There also exists a caspase-independent apoptotic pathway that is mediated by AIF (apoptosis-inducing factor).

The term "autophagy" (or autophagocytosis) means the basic catabolic mechanism that involves cell degradation of unnecessary or
dysfunctional cellular components through the actions of lysosomes.

The breakdown of cellular components promotes cellular survival during starvation by maintaining cellular energy levels. Autophagy allows the degradation and recycling of cellular components. During this process, targeted cytoplasmic constituents are isolated from the rest of the cell within a double-membrane vesicle known as an autophagosome. The autophagosome then fuses with a lysosome and its cargo is degraded and recycled. There are three different forms of autophagy that are commonly described: macroautophagy, microautophagy and chaperone-mediated autophagy.

In the context of disease, autophagy has been seen as an adaptive response to stress promoting survival, whereas in other cases it appears to promote cell death and morbidity.

One of the mechanisms of programmed cell death (PCD) is associated with the appearance of autophagosomes and depends on autophagy proteins. This form of cell death most likely corresponds to a process that has been morphologically defined as autophagic PCD.

A first aspect of the present invention that is as defined in the claims refers to an antibody able to recognize and bind to the ectodomain of the NG2/CSPG4 transmembrane proteoglycan. Applicants have found that the antibody induces cancer cell apoptosis upon high- affinity binding to its antigen. In particular, the antibodies of the invention recognize and bind with high-affinity and high selectivity discrete NG2/CSPG4 isoforms or the proteolitically processed forms of the protein.

The NG2/CSPG4 isoform, or the proteolitically processed form of the protein to which the present invention refers to has a molecular weight ranging from 50-280 kDa, preferably from 110-260 kDa, more preferably from 120 to 250 kDa.

Therefore, the present invention refers to an apoptosis inducing antibody able to recognize and bind with high-affinity and high selectivity NG2/CSPG4, in particular discrete NG2/CSPG4 isoforms. In other words, after binding the ectodomain of the cognate NG2/CSPG4 antigen expressed on cancer cells, the
antibody of the invention activates at least one of the pathways above known to cause and/or be involved in the execution of programmed cell death.

The antibody of the invention is preferably a monoclonal antibody.

The antibody of the invention recognizes and binds with high-affinity a defined portion of the ectodomain of NG2/CSPG4, preferably present in discrete NG2/CSPG4 isoforms, said ectodomain of NG2/CSPG4 being preferably SEQ ID NO: 17, corresponding to the NCBI Reference Sequence NC 000015.10.

As already stated, NG2 is the orthologous gene of human CSPG4 in rat. NG2/CSPG4 is expressed on cancer cells and angiogenic vasculature, and its expression is associated with an aggressive disease course in several malignancies. Alternative names of NG2/CSPG4 are High Molecular Weight Melanoma-Associated Antigen (HMW-MAA), Melanoma Cell Surface Proteoglycan (MCSP, MCSPG) and melanoma proteoglycan (Mel-PG).

Preferably, Human CSPG4 gene corresponds to the NCBI Reference Sequence: NM_001897.4.

Preferably, NG2 gene corresponds to the NCBI Reference Sequence: NM_031022.1.

According to a preferred embodiment of the present invention the antibody comprises:
- SEQ ID NO: 18, 19 and SEQ ID NO: 20, 21, 22, wherein SEQ ID NO: 18, 19 are respectively CDR1 and CDR2 of the heavy chain of the antibody, and SEQ ID NO: 20, 21, 22 are respectively CDR1, CDR2 and CDR3 of the antibody, wherein the antibody is preferably produced by the hybridoma cell line 2161D2; and/or
- SEQ ID NO: 23, 24 and SEQ ID NO: 25, 26, 27, wherein SEQ ID NO: 23, 24 are respectively CDR1 and CDR2 of the heavy chain of the antibody, and SEQ ID NO: 25, 26, 27 are respectively CDR1, CDR2 and CDR3 of the antibody, wherein the antibody is preferably produced by the hybridoma cell line 2164H5; and/or
- SEQ ID NO: 28, 29, 30 and SEQ ID NO: 31, 32, 33, wherein SEQ ID NO: 28, 29, 30 are respectively CDR1, CDR2 and CDR3 of the heavy chain of the antibody, and SEQ ID NO: 31, 32, 33 are respectively CDR1, CDR2 and CDR3 of the antibody, wherein the antibody is preferably produced by the hybridoma cell line 2166G4; and/or
- SEQ ID NO: 34, 35, 36 and SEQ ID NO: 37, 38, 39, wherein SEQ ID NO: 34, 35, 36 are respectively CDR1, CDR2 and CDR3 of the heavy chain of the antibody, and SEQ ID NO: 37, 38, 39 are respectively CDR1, CDR2 and CDR3 of the antibody, wherein the antibody is preferably produced by the hybridoma cell line 2172B12.

According to a preferred embodiment of the present invention the antibody has a variable heavy chain (VH) selected from the group consisting of: SEQ ID NO: 1, 5, 9 and 13.

According to a preferred embodiment of the present invention the antibody has a variable light chain (LH) selected from the group consisting of: SEQ ID: 3, 7, 11 and 15.

According to a preferred embodiment the antibody has a VH selected from the group consisting of: SEQ ID NO: 1, 5, 9 and 13 or the corresponding nucleic sequences SEQ ID NO: 2, 6, 10 and 14 and a LH selected from the group consisting of: SEQ ID: 3, 7, 11 and 15.

According to a preferred embodiment of the present invention, the antibody has SEQ ID NO: 1 as VH and SEQ ID NO: 3 as VL.

According to a preferred embodiment of the present invention, the antibody has SEQ ID NO: 5 as VH and SEQ ID NO: 7 as VL.

According to a preferred embodiment of the present invention, the antibody has SEQ ID NO: 9 as VH and SEQ ID NO: 11 as VL.

According to a preferred embodiment of the present invention, the antibody has SEQ ID NO: 13 as VH and SEQ ID NO: 15 as VL.

In a preferred form of the invention, the monoclonal antibody has preferably the VH and VL of the murine hybridoma cell line deposited at International Depositary Authority of Canada with the Accession Number 121214-01.

In a further preferred form of the invention, the monoclonal antibody has preferably the VH and VL of the murine hybridoma cell line deposited at International Depositary Authority of Canada with the Accession Number 121214-02.

In a further preferred form of the invention, the monoclonal antibody has preferably the VH and VL of the murine hybridoma cell line deposited at International Depositary Authority of Canada with the Accession Number 121214-03.

In a further preferred form of the invention, the monoclonal antibody has preferably the VH and VL of the murine hybridoma cell line deposited at International Depositary Authority of Canada with the Accession Number 121214-04.

The hybridoma having the Accession Number 121214-01 produces the antibody here disclosed as clone 2166G4/C10/D1 (Clone 2166G4).

Preferably, the antibody 2166G4/C10/D1 has SEQ ID NO: 13 as VH and SEQ ID NO: 15 as VL or the corresponding nucleic sequence SEQ ID NO: 14 and 16.

The hybridoma having the Accession Number 121214-02 produces the antibody here disclosed as clone 2172B12/C10 (Clone 2172B12).

Preferably, the antibody 2172B12/C10 has SEQ ID NO: 9 as VH and SEQ ID NO: 11 as VL or the corresponding nucleic sequence SEQ ID NO: 10 and 12.

The hybridoma having the Accession Number 121214-03 produces the antibody here disclosed as clone 2164H5/E11 (clone 2164H5).

Preferably, the antibody 2164H5/E11 has SEQ ID NO: 5 as VH and SEQ ID NO: 7 as VL or the corresponding nucleic sequence SEQ ID NO: 6 and 8.

The hybridoma having the Accession Number 121214-04 produces the antibody here disclosed as clone 2161D2/C2 (clone 2161D2).

Preferably, the antibody 2161D2/C2 has SEQ ID NO: 1 as VH and SEQ ID NO: 3 as VL or the corresponding nucleic sequence SEQ ID NO: 2 and 4.

The sequences relevant for the scope of the present invention are summarized in Table I. The sequences of the invention are disclosed according to the international standard WIPO ST.25 and the description thereof was developed by using the program Patent-In 3.5.

Sequence description is reported as follows.

Table I shows all the amino acid and nucleotide sequences and the corresponding "Sequence Identifiers" defining the antibodies disclosed in the present invention.

Also contemplated are all the nucleic sequences derived from the nucleotide sequences shown in Table I because of the genetic code degeneration.

**Table I**

| | | |
|---|---|---|
| Heavy chain variable region sequence of 2161D2 clone antibody (Protein) | | SEQ ID NO: 1 |
| Heavy chain variable region sequence of 2161D2 clone antibody (DNA) | | SEQ ID NO: 2 |
| Light chain variable region sequence of 2161D2 clone antibody (Protein) | | SEQ ID NO: 3 |
| Light chain variable region sequence of 2161D2 clone antibody (DNA) | | SEQ ID NO: 4 |
| Heavy chain variable region sequence of 2164H5 clone antibody (Protein) | | SEQ ID NO: 5 |
| Heavy chain variable region sequence of 2164H5 clone antibody (DNA) | | SEQ ID NO: 6 |
| Light chain variable region sequence of 2164H5 clone antibody (Protein) | | SEQ ID NO: 7 |
| Light chain variable region sequence of 2164H5 clone antibody (DNA) | | SEQ ID NO: 8 |
| Heavy chain variable region sequence of 2172B12 clone antibody (Protein) | | SEQ ID NO: 9 |
| Heavy chain variable region sequence of 2172B12 clone antibody (DNA) | | SEQ ID NO: 10 |
| Light chain variable region sequence of 2172B12 clone antibody (Protein) | | SEQ ID NO: 11 |
| Light chain variable region sequence of 2172B12 clone antibody (DNA) | | SEQ ID NO: 12 |
| Heavy chain variable region sequence of 2166G4 clone antibody (Protein) | | SEQ ID NO: 13 |
| Heavy chain variable region sequence of 2166G4 clone antibody (DNA) | | SEQ ID NO: 14 |
| Light chain variable region sequence of 2166G4 clone antibody (Protein) | | SEQ ID NO: 15 |
| Light chain variable region sequence of 2166G4 clone antibody (DNA) | | SEQ ID NO: 16 |
| NG2/CSPG4 ectodomain | | SEQ ID NO: 17 |

A further aspect of the present invention refers to the antibody of the invention, embodied in a bispecific antibody construct (e.g. an
antibody binding both NG2/CSPG4 and another cell surface molecule and/or antigen) or a fusion protein comprised of a therapeutic amount of the antibody and an another therapeutic molecule, which is preferably a cytokine or an aptoptosis-inducing agent (e.g TRAIL or Fas Ligand), or a CAR T (Chimeric Antigen Receptor T cell) construct in which an scFv
of the antibody of the invention is expressed on the surface of a CAR T cell, such as to simultaneously engage NG2/CSPG4 expressing cancer cells and endogenous T cells and favor the maintenance of the CAR T cell in proximity to the cancer cell to trigger the activation of the engaged T cell. The invention further describes an expression vector comprising the nucleotide sequences here disclosed. Said expression vector comprises
all the nucleotide sequences requested for the antibody expression in a host cell. Described herein, but not part of the invention, is a host cell comprising said expression vector.

The sequences necessary for expression in prokaryotes regard for example a promoter and, optionally, an operator sequence, a ribosome-binding site and possibly other sequences. For eukaryotic cell expression, sequences such as promoters, enhancers, termination signals and polyadenilation are required.

Said expression vector may also comprises signal sequences for targeting the antibody or its variant disclosed above in a particular cellular compartment.

Said vector may further comprise any selection gene whose expression is easily used for selecting the recombinant host cells transformed with the vector.

A further aspect of the present invention refers to a composition comprising the antibody disclosed above and pharmaceutically acceptable excipient generally used for this purpose.

The antibody, or the composition of the invention is preferably used for treating a disease whose treatment relies upon external induction of apoptosis in the cells responsible for the disease. In other words, the invention contemplates any disease for which programmed

cell death, such as apoptosis, induced by external means can effectively be exploited for curative purposes.

Described herein, but not part of the invention, is a method for treating a disease dependent upon resistance to programmed cell death. It involves a step of administration to an individual, suspected to be or affected by such disease, of an effective amount of the antibody or the composition of the invention. The administration to the individual allows the binding of the administered antibody to the NG2/CSPG4 ectodomain expressed on the membrane of the cells responsible, or associated with the disease to be treated.

Preferably, the antibody or the composition is administered systemically or locally, more preferably by injection, or by any other means allowing the antibody to most effectively reach the target cells.

For the scope of the present invention, the disease of interest is preferably cancer, primarily solid, hematological malignancies or any other diseases requiring a curative elimination of cells expressing NG2/CSPG4 are also contemplated.

More preferably, but not exclusively, said disease is cancer, preferably a cancer selected from the group consisting of: any variant, subtype or histotype of melanomas, soft-tissue, cartilage or bone sarcomas, head or neck carcinomas, colorectal carcinomas, lung carcinomas, prostate carcinomas, breast carcinomas, skin carcinomas, mesotheliomas, hematological neoplasia or Central Nervous System (CNS) and Peripheral Nervous System (PNS) tumors.

The antibody or the composition is preferably used for treating and/or preventing formation of secondary metastatic and non-metastatic lesions, and relapsing local, loco-regional and distant tumor formations.

The antibody of the invention functions by recognizing and binding with high-affinity the NG2/CSPG4 transmembrane PG, preferably discrete NG2/CSPG4 isoformes, expressed by cells and by activating in these cells defined programmed cell death pathways. These pathways drive the cell to

its death by apoptotic, preferable caspase-dependent, and autophagic mechanisms, or related mechanisms of programmed cell death, in a manner apparently independent of other intervening extracellular factors.

According to a preferred embodiment, the antibody or the composition of the invention is administered (used) in
combination with other drugs, preferably with other conventional or experimental targeted and non-targeted chemotherapeutic agents. Alternatively, the antibody or the composition of the invention stands alongside other therapeutic treatments, such as surgical resection and/or radiotherapy.

Being NG2/CSPG4 expression on cancer cells putatively associated with cancer insurgence, progression and recurrence, a further aspect of the present invention is the use of the antibody of the invention for diagnostic use, preferably as a biomarker for the routine clinical monitoring of cancer patients and/or for in vivo whole-body or local diagnostic imaging procedure and/or for identification, enumeration and isolation of cancer circulating cells.

Also described herein, but not part of the invention, is a naked polypeptide formulation of the naturally produced and/or humanized and/or genetically engineered and/or recombinantly produced and/or synthetically derived compound active in inducing cell death and including at least one of the anti-NG2/CSPG4 antibody, or fragments thereof, of the invention.

### EXAMPLE

In the present invention, four murine monoclonal antibodies were produced by using a recombinant, eukaryotic fragment corresponding to the extracellular portion of the NG2/CSPG4 transmembrane proteoglycan and by adopting the conventional Koehler and Milstein immunization and hybridoma production method (Harlow E. and Lane D., "Antibodies: a laboratory manual", Cold Spring Harbor Laboratory, 1988; Howard G.C. and Kaser M.R., "Making and using antibodies: a practical handbook", CRC Press Taylor & Francis Group, 2006)

### Isotyping of the NG2/CSPG4 monoclonal antibodies

In order to identify classes, subclasses, and type of light chains that belong to immunoglobulins produced by hybridomas, Pierce® Rapid ELISA Mouse antibody Isotyping Kit was used. This assay uses ELISA strip-well plates with individual wells, pre-coated with either anti-mouse heavy-chain capture antibody (anti-IgG1, IgG2a, IgG2b, IgG3, IgA and IgM), or anti-mouse light-chain capture antibody (kappa or lambda). This approach eliminates the need to purify and immobilize an antigen for isotyping.

Supernatants of hybridoma cells grown in Ig-depleted serum were diluted 1:10 in TBS (Tris-buffered saline) and dispensed into the plates. Antibody binding was detected using polyvalent secondary HRP-conjugated polyclonal antibodies, and the plates were incubated at room temperature under shaking for 1 hour in the dark. Subsequently, wells were thoroughly rinsed with wash buffer, and then incubated with 3,3',5,5'-Tetramethylbenzidine (TMB) Liquid substrate (Sigma-Aldrich) for 10 min and absorbance of the colorimetric reaction was measured at 450 nm using a microplate adsorbance reader.

### All antibodies were found of the IgG1 class.

### Antibody binding to putative NG2/CSPG4 isoforms as evidenced by cell-ELISA and flow cytometry

In order to evaluate the ability of the antibodies to recognize diverse NG2/CSPG4 isoforms they were initially assayed on a panel of NG2/CSPG4-expressing cells by using a cell-ELISA approach on live and fixed cells, followed up by conventional flow cytometry. The cellular models used for these experiments were the following cell lines: SK-LMS1 and SK-UT-1 (human leiomyosarcoma cell lines), HT1080^{NG2+} (human fibrosarcoma cell line immunosorted by FACS using the commercially available pan-anti-NG2/CSPG4 antibody 9.2.27), 143B (human bone osteosarcoma), A375 (human melanoma) and M2 (human melanoma). For cell-ELISA on fixed cells, test cells were seeded in 96 multiwell plates in complete growth medium (10,000 cells/well). After fixation with 2% PFA for 30 min, endogenous peroxidase activity was neutralized by incubation with 3% H₂O₂ for 15 min at room temperature. Blocking of non-specific binding sites was performed by further incubation of the cells with blocking buffer (PBS with 2% BSA; 10% sucrose; 0,1% NaN₃) for 30 min at room temperature. Cells were finally incubated overnight at 4°C with the anti-NG2/CSPG4 antibodies, diluted as follows: supernatants, 1:1-1:2 and ascites fluids, 1:50 in blocking buffer. The Streptavidin/Biotin (Thermo Scientific TS-125-HR and TM-125-BN) and TMB (T4444 Sigma) detection method was used for the detection of the binding of the antibodies to the antigen, according to the procedure recommended by the manufacturer. All assays were performed in triplicate.

For cell-ELISA on live cells, test cells were seeded in 96 multiwell plates in complete growth medium (melanoma cells at 25,000 cells/well; sarcoma cells at 20,000 cells/well). Blocking of non-specific binding sites on cells was performed by incubating them with sterile blocking buffer (PBS and 2% BSA; 10% sucrose) for 30 min at 37°C. Then, cells were further incubated for 2 hours at 37°C with the anti-NG2/CSPG4 antibodies, diluted as follows: supernatants, 1:1-1:5 and ascites fluids, 1:50-1:100 in blocking buffer. After fixation with 2% PFA for 30 min, endogenous peroxidase activity was quenched by incubation with 3% H₂O₂ for 15 min at room temperature. Detection of bound antibody was similarly detected through the Streptavidin/Biotin (Thermo Scientific TS-125-HR and TM-125-BN) and TMB (T4444 Sigma) system described above. All assays were performed in triplicate. The outcome of these experiments is summarized in Table 2. Antibodies derived from the same immunization and spleen fusion protocol, but found to be did not react with certain cell lines, these cases were adopted as internal negative controls.

**Table 2**

| | cell-ELISA (fixed cells) | | | | cell-ELISA (live cells) | | | |
|---|---|---|---|---|---|---|---|---|
| CLONE | A375 | SK-UT1 | 143B | M2 | A375 | SK-UT1 | SK-LMS1 | M2 |
| 2161D2/C2 | 11 | 1 | 9 | 0 | 42 | 15 | 25 | 0 |
| 2164H5/E11 | 0 | 61 | 21 | 0 | 5 | 20 | 0 | 0 |
| 2166G4/C10/D1 | 40 | 37 | 73 | 0 | 35 | 17 | 38 | 0 |
| 2172B12 | 4 | 15 | 0 | 0 | 31 | 57 | 5 | 0 |

Each antibody was found to exhibit a different reactivity pattern, supporting the idea that it recognizes a distinct NG2/CSPG4 isoform. In addition, to note was that antibodies display different binding patterns when tested on live or fixed cells, which further indicates that they possess diverse affinities for native and partly or entirely denatured forms of NG2/CSPG4.

To then confirm by a different method the relative expression of NG2/CSPG4 isoforms detected by the antibodies on tumor cells, flow cytometric analyses were performed using the commercially available pan-human NG2/CSPG4 monoclonal antibody 7.1 PE-labeled (Beckman-Coulter) as reference. While untreated cells and cells treated with non-specific immunoglobulines were used as a negative control.

Cells were detached with Accutase or EDTA, collected in tubes and counted. A minimal amount of 300,000 cells for was used for each antibody or control sample. Cells were rinsed with PBS (with 1% FBS) by centrifugation at 0.3 RCF, then they were incubated with antibodies in the dark at 4 °C for 30 minutes. After antibody incubation, cells were rinsed twice with PBS by centrifugation at 0.3 RCF, and resuspended in PBS (with 1% FBS). All samples were analyzed using a FACScan flow cytometer (BD Biosciences) and the resulting bindings analysed using the flow cytometer software BD Cell Quest ProTM. Relative NG2/CSPG4 expression on the different tumor cell lines is summarized in Table 3.

**Table 3**

| | NG2/CSPG4 expression (%) | | | | | | |
|---|---|---|---|---|---|---|---|
| CLONE | SK-LMS1 | SK-UT1 | COLO38 | HT1080^{NG2+} | A375 | M2 | 143B |
| 2161D2/C2 | 3 | 12 | 19 | 0 | 74 | 0 | 86 |
| 2164H5/E11 | 9 | 1 | 11 | 92 | 89 | 0 | 57 |
| 2166G4/C10/D1 | 3 | 3 | 48 | 26 | 71 | 0 | 0 |
| 2172B12/C10 | 64 | 47 | 16 | 94 | 91 | 0 | 32 |
| 7.1-PE (control) | 89 | 64 | 94 | 38 | 92 | 96 | 62 |

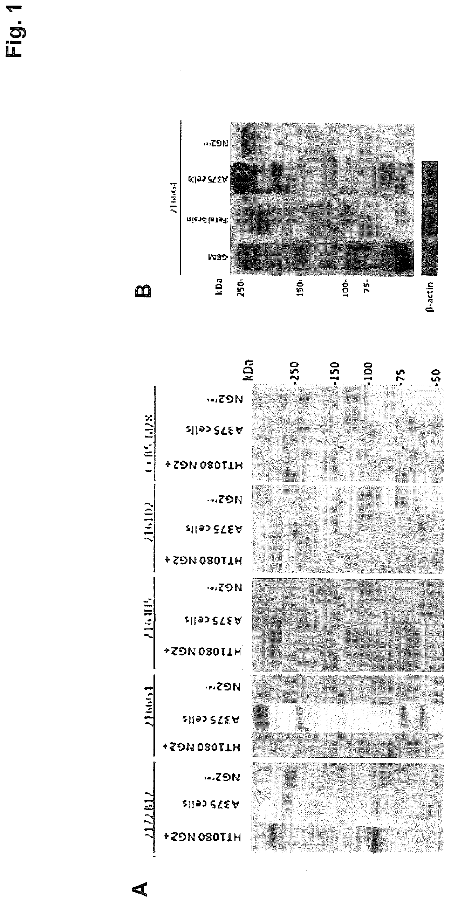

The outcome of these experiments further corroborated a putative expression of different antibody-reactive isoforms on tumor cells and specifically confirmed that M2 melanoma cells express a NG2/CSPG4 isoform(s) not recognized by these reagents. Differential expression of NG2/CSPG4 isoforms on the model cell lines, and the extracellular proteolytic processing of these isoforms, was eventually also investigated by immunoblotting using lysates of A375 and HT1080^{NG2+} cells (Fig.1A). For this purpose, cells were directly lysed in sample buffer and the lysates resolved by SDS-PAGE under reducing conditions on 5% or 4-15% gradient gels, followed by transferring onto nitrocellulose membranes and incubation with the indicated anti-NG2/CSPG4 antibodys. Recombinant NG2/CSPG4 (NG2^{rec}) was used as reference. In these experiments, NG2/CSPG4 isoforms and their proteolytic fragments were also studied on lysates of pericyte sprouts of fetal brain neovessels and in glioblastoma multiforme lesions (GMB; Fig.1B). Sections from brain areas taken adjacently to sections with abundant angionenesis and enrichment of NG2/CSPG4-expressing pericytes. Immunoblotting of β-actin with a commercial polyclonal antibody was used for normalization of the gel lane loading.

The banding pattern observed for A375 cells indicated that these cells express mainly two NG2/CSPG4 isoforms: one an apparent MW greater than 250 kDa and the other of a smaller size. These isoforms were primarily recognized by antibodies 2166G4/C10/D1 and 2164H5/E11. Conversely, antibodies 2172B12/C10 and 2161D2/C2 only recognized the smaller isoform expressed by A375 cells. HT1080^{NG2+} cells did not seem to express the smaller isoform, but displayed the larger isoform recognized by antibodies 2172B12/C10 and 2164H5/E11. Antibody 2161D2/C2 was found to recognize preferentially a proteolytically processed form(s) of the protein running between 100-120 kDa, while antibody 2172B12/C10 showed a broader isoform reactivity documented by binding to different isoforms and their proteolytic fragments which had apparent MW is in the range of 75 to 250 kDa. Fetal brain NG2/CSPG4 isoforms were poorly recognized and it seemed that antibody 2166G4/C10/D1 was the one that in this tissue recognized the primary isoforms and their proteolytic fragments (Fig. 1B).

These immunochemical analyses corroborate the distinct molecular identity of the NG2/CSPG4 isoforms recognized by the four antibodies and further indicate that some of them may specifically detect naturally occurring proteolytic forms of the proteoglycan generated at the time or following cell surface shedding of its extracellular portion. Subcellular localization of NG2/CSPG4 isoforms detected by the antibodies as determined by in vitro immunolabeling
Tumor cell lines A375, Hs578T (human breast carcinoma) and HT1080^{NG2+} and primary human brain vascular pericytes (HBVP) were grown to subconfluence and stained with the different antibodies. For this purpose, 25,000 cells were seeded into each well of a 24-well plate, in which a round glass coverslip had been placed at the bottom. Tumor cell lines were routinely grown in DMEM medium supplemented with 10% FBS, whereas HBVP were cultured in PM medium supplemented with 1% penicillin/streptomycin, 1% PGS and 2% FBS as recommended by the supplier (Lonza). After 24 hours of culture, cells were fixed at room temperature with 4% PFA for 20 min and non-specific binding sites were blocked by incubation with 20% Normal Goat Serum (NGS) in PBS for 30 min at room temperature. Cells were then incubated overnight at 4° C with antibody supernatants diluted 1:1-1:2 in NGS, or purified antibodies used at 150-300 ng/µl. After washes, coverslips were incubated for 1 hour at room temperature with a goat anti-mouse secondary antibody conjugated to the Alexa Fluor® 594 dye diluted 1:250 in PBS. Next, they were washed and incubated for 5 minutes with Hoechst (Sigma Aldrich) diluted 1:1,000 in PBS. Coverslips were finally washed and mounted on microscope slides with Non-Fluorescing Mounting Aqua-Poly/Mount (Polisciences, Inc) and viewed under a fluorescence microscope using appropriate a filter sets.

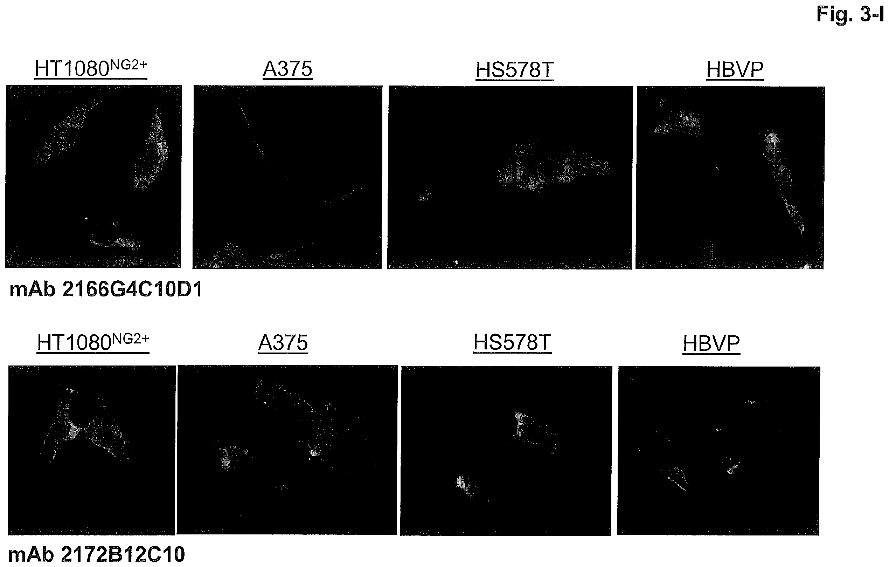

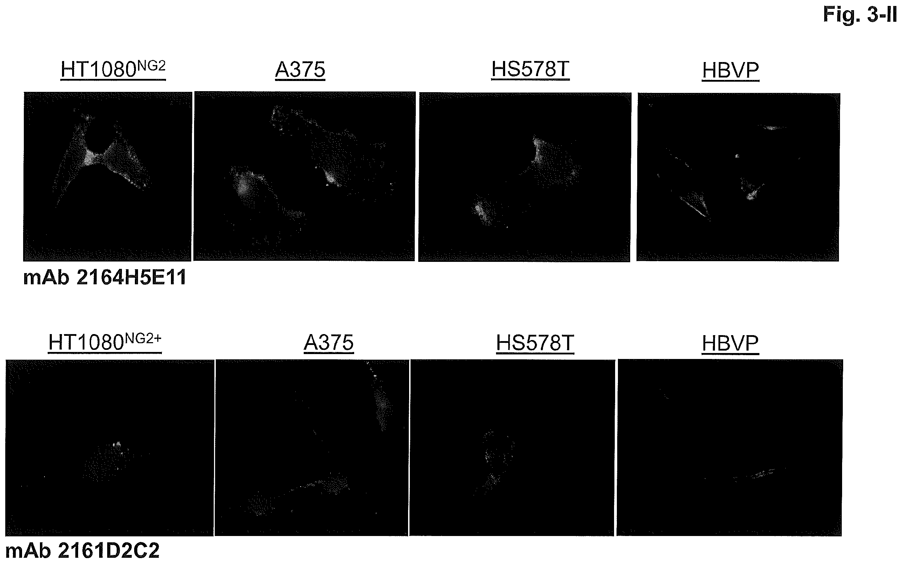

As expected, NG2/CSPG4 was primarily immunolocalized on the cell membrane of both tumor cell lines, while the different staining intensity is believed to reflect the efficiency with which each antibody detected the corresponding isoform in that specific cellular compartment. In some cases, staining for NG2/CSPG4 was also observed in intracellular locations, suggesting that some of the antibodies recognized the nascent NG2/CSPG4 polypeptide and may additionally have identified spontaneously internalized NG2/CSPG4 molecules (Fig 3I-II).

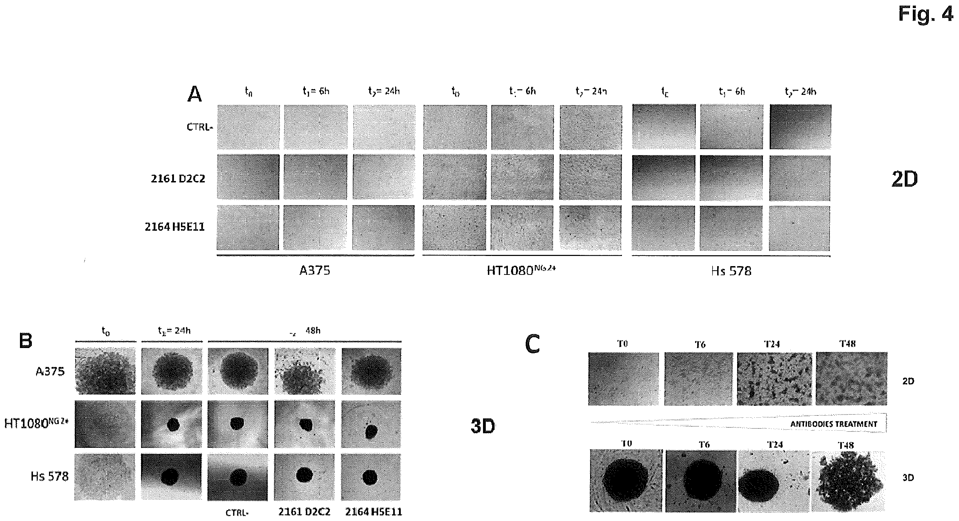

Effects of anti-NG2/CSPG4 antibodies on cell-substrate adhesion and migration in vitro. To evaluate the ability of the antibodies to neutralize the function of NG2/CSPG4 in the control of cellular interactions, with particular reference to the role exerted by the proteoglycan in the regulation of cell-substrate adhesion and cell motility, the above cell lines were incubated with the antibodies and monitored by microscopy in 2D monolayer culture of as 3D tumor spheroids generated by prior culture of the cells on Poly-HEMA substrates. Cell motility studies were carried out adopting the conventional scratch-based wound-healing type assay as a paradigm. In all these experiments and the assays described below, we routinely used anti-NG2/CSPG4 antibodies derived from the same immunization and spleen fusion protocol and possessing either a marked ability to react with the native antigen expressed on the cell surface or lacking such ability, but which had not been found to affect cell behavior in pilot tests. For these experiments the above described melanoma, fibrosarcoma and breast carcinoma cell lines were seeded in 24 well plates and examined at 6, 24, 48 hours time intervals, with and without addition of purified anti-NG2/CSPG4 antibodies administered at 20-30 ng/ml in serum-free growth medium. After microscopic end-point evaluation, cells were lysed and processed for immunoblotting with antibodies against cytoskeletal components and antibodies to cell death markers (see below). Treatment of cells with antibodys 2161D2/C2 and 2164H5/E11 caused cell shape changes and detachment of the cells within 24 hours both in 2D (Fig. 4A-5A) and in spheroids 3D settings (Fig. 4B-5B).

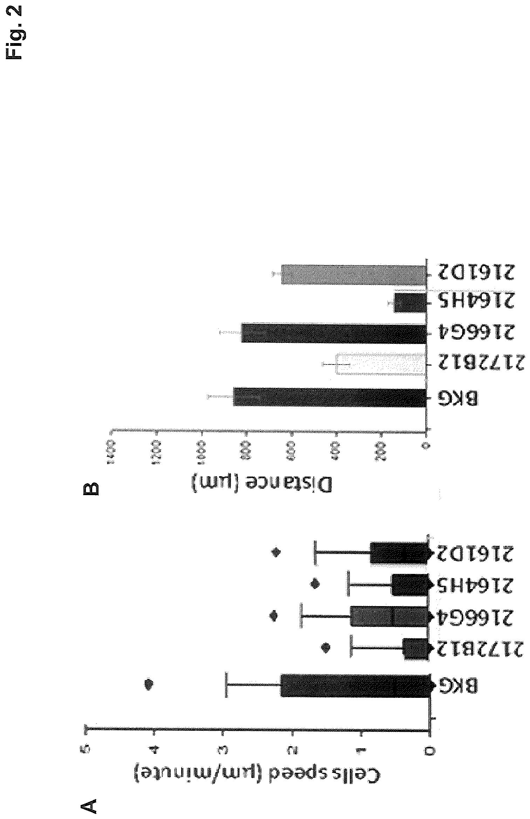

For cell migration assays, HT1080^{NG2+} cells were selected as a preferential model because of their pronounced locomotory abilities. Confluent cells were starved and then scratched in the middle of the monolayer and exposed to diverse quantities of the purified anti-NG2/CSPG4 antibodies as described above. Cell migration was monitored by phase-contrast video time-lapse microscopy for 24 hours. Cell migration rate was assessed by mean displacement (MD, i.e. the mean distance (mm) traveled per minute) of the cell centroid. The migratory potential of cell population was then be expressed as the average mean displacement (AMD) of all cells in the analysis. In order to determine how the manual selection of the centroid positions of cells influences the calculated migration rates of individual cells and cell populations, cells were manually marked by using a point-and-click system. In this manual cell tracking method, a subset of cells from each time-lapse video was selected for analysis. This subset is assumed to represent the whole cell population. The results are summarized in Fig. 2. In the first histogram (Fig. 2A) cells speed for each different well is plotted; in the second histogram (Fig. 2B) the covered distances is plotted for each antibody. The results show that anti-NG2/CSPG4 antibodies are able to differentially interfere with cell motility, an effect that was particularly evident for antibodies 2164H5/E11 and 2172B12/C10. Incubation of NG2/CSPG4 expressing cells with the antibodies induces programmed cell death.

The observed changes in cell shape observed after incubation of cells with the anti-NG2/CSPG4 suggested that engagement of the proteoglycan with these specific antibodies could uniquely induce intracellular alterations triggering programmed cell death. To verify this possibility A375 and HT1080^{NG2+} cells were time- and dose-dependently treated with the four antibodies of this invention and examined through a proprietary multiparametric cell death detection platform. Staurosporine was used at a concentration of 500 nM as positive control. Preliminary screening experiments established that purified antibodies were most effective at a concentration of 20-30 ng/µl and were therefore consistently used at this concentration. To effectively detect apoptotic cells, we initially used the DeadEnd™ Fluorometric TUNEL Assay combined with PI staining. For this purpose, cells cultivated on coverslips within 24 well plates were fixed with 4% PFA for 20 min at room temperature, followed by extensive washing with PBS and permeabilization with 0.1% Triton X-100 in PBS for 10 minutes. Equilibration Buffer was then added at room temperature for 5-10 minutes was added in each well and cells were further incubated with Equilibration Buffer supplemented with Nucleotide Mix and rTdT Enzyme at 37°C for 60 min inside a humidified chamber to allow the tailing reaction to occur. Incubation buffer was then removed and 2X SSC (Saline Sodium Citrate) was added for 15 min at room temperature. The samples were finally extensively with PBS to remove unincorporated fluorescein-12-dUTP and incubated with PI (1µg/ml in PBS) for 15 minutes at room temperature in the dark. Coverslips were removed from the wells washed with deionized water for 5 minutes at room temperature, mounted on microscope slides and viewed under a fluorescence and confocal laser microscopy using the appropriate filter sets.

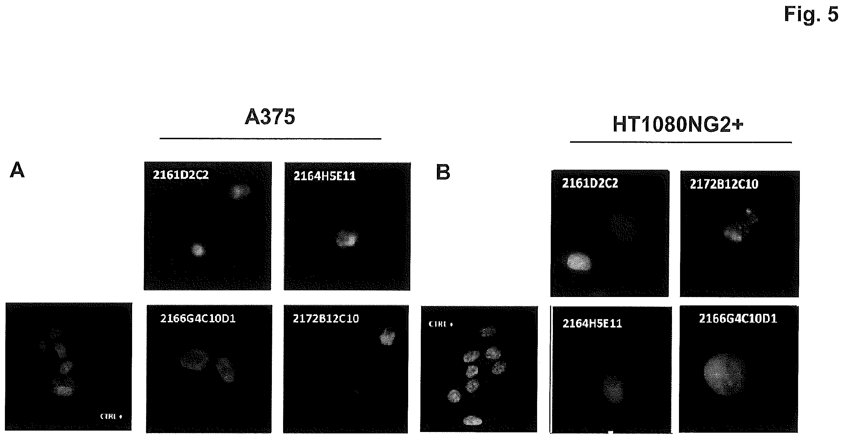

The results reported in Fig. 5 document the differentially ability of the antibodies to induce programmed cell death in the two cell lines.

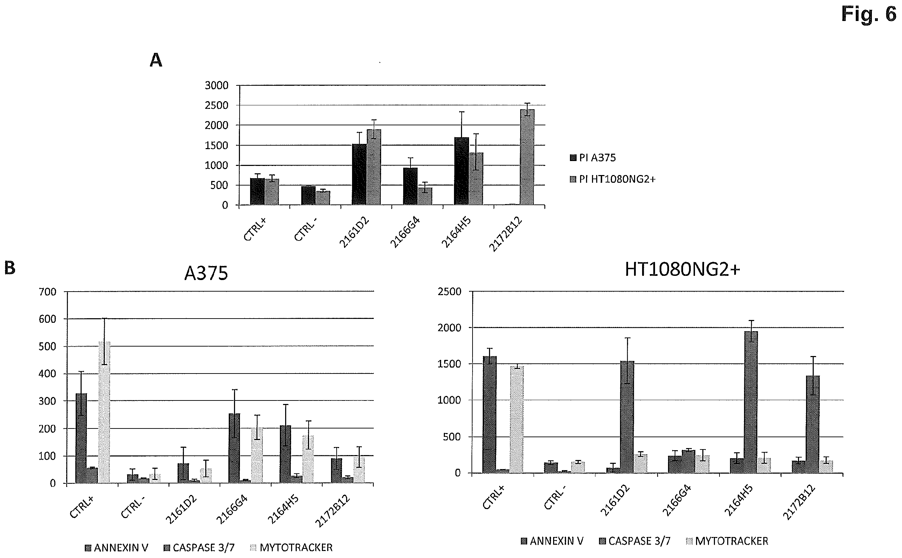

These experiments were also repeated using the Acumen eX3 microplate laser cytometer (TTP Labtech Ltd, UK). When comparing to the control, the NG2/CSPG4-specific antibodvs 2161D2C2 and 2164H5E11 increased by a three-fold the number of apoptotic A375 and HT1080^{NG2+} cells; whereas antibody 2166G4/C10/D1 had no effect on HT1080^{NG2+}, but doubled the A375 apoptotic cells number. Antibody 2172B12/C10 promoted full apoptosis in HT1080^{NG2+}, but not A375 cells (Fig. 6).

To further examine the extent to which the different antibodies were able to promote the different phases of apoptosis we applied the multiparametric assay additionally involving detection of phosphatidylserine translocation through APC-tagged Annexin V and mitochondrial membrane potential changes assessed through the Mytotraker Red marker (Life Technologies, Inc.). In the same multiparametric assay, the activation of Caspase 3/7 was also investigated by CellEventTMCaspase-3/7 Green Detection Reagent (Fig. 6).

We observed that the NG2/CSPG4-specific antibodys 2161 D2/C2 had a weak effect, whereas antibodys 2166G4/C10/D1 and 2164H5/E11 doubled the apoptotic A375 cells compared to the control. All antibodys were effective on HT1080^{NG2+} cells, in particular when evaluating caspase activation, although 2166G4/C10/D1 showed the weakest action.

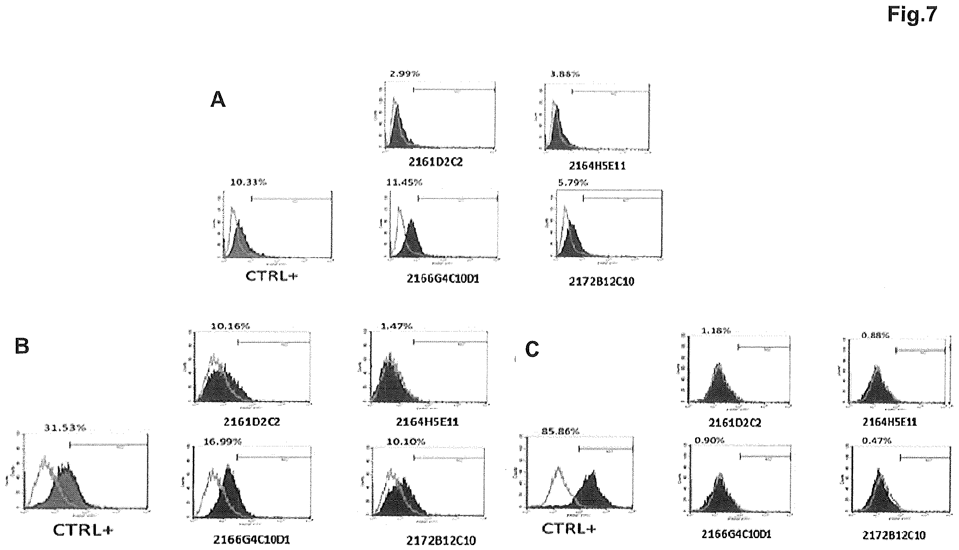

We next added another apoptotic marker to the system and assessed PARP cleavage by flow cytometry. To this end, A375 (Fig. 7A), HT1080^{NG2+} (Fig. 7B), and Hs578T (Fig. 7C) cells, cultured as monolayers, were treated with anti-NG2/CSPG4 antibodies as described above and examined by flow cytometry with an antibody specifically recognizing cleaved PARP. These experiments were complemented by Western blotting experiments using analogous antibodies directed against cleaved PARP. The results of these experiments indicate a stronger apoptosis induction in HT1080^{NG2+} and A375 cell lines after treatment with 2166G4/C10/D1 compared to that seen in Hs578T cells.

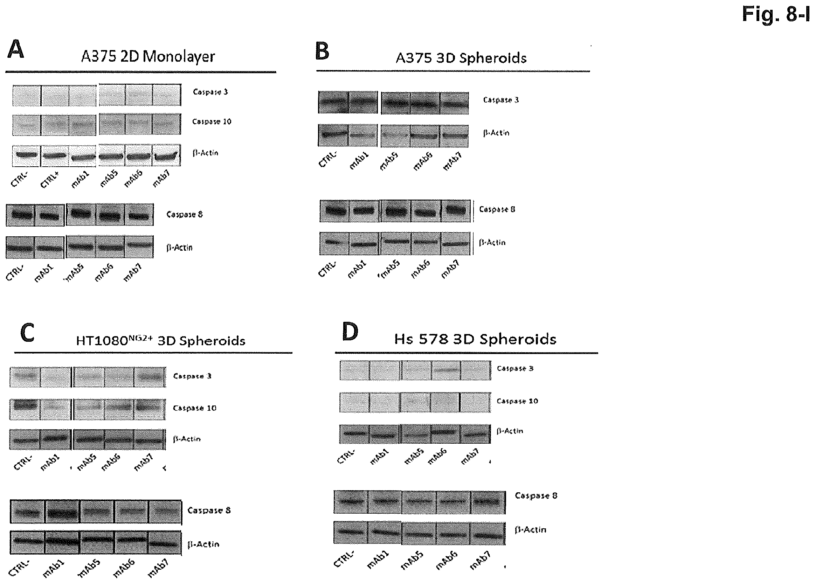

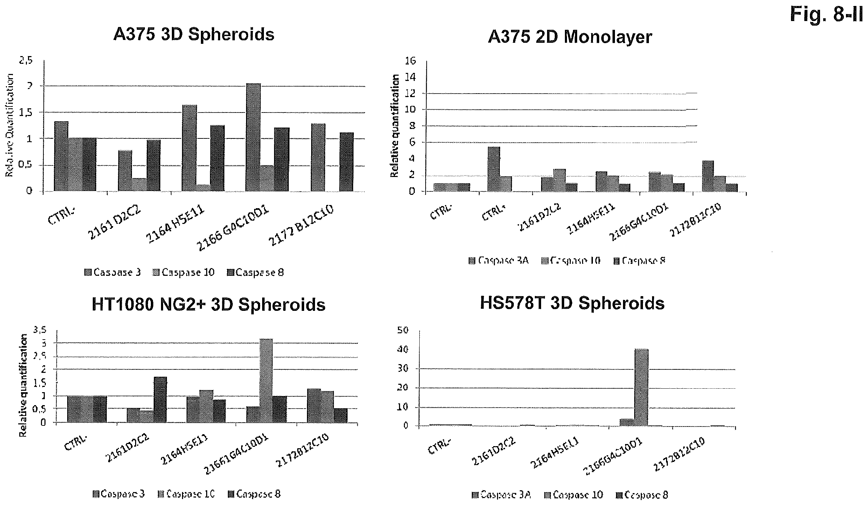

Corroborative, parallel detection of activated effector caspases was performed by immunoblotting on lysates from cells treated with antibodies in 2D monolayer and 3D multicellular spheroids settings. In these cases cell lysates were resolved by SDS-PAGE under reducing conditions, followed by transferring onto nitrocellulose membranes and immunoblotting with antibodies against pro-caspase forms and the activated forms of caspases 3, 8 and 10. Antibodies to β-actin were used as internal normalization standard. Band intensities were analyzed through densitometry and normalized for the internal standard. Relative caspase activation was assessed by establishing the ratio between sample bands intensities and negative control. The results of these experiments are reported in Fig. 8 where it is evident that the anti-NG2/CSPG4 antibody displaying the greatest pro-apoptotic effect documented by caspase activation was antibody 2172B12/C10. In A375 cells cultured as 3D multicellular spheroids, all antibodies induced cleavage of both caspase 3 and 8 (Fig. 8 I-II). Conversely, caspase 10 seemed to be the primary caspase to be activated in HT1080^{NG2+} cells grown as 3D multicellular spheroids (Fig. 8 I-II), and the most effective antibody in this case was 2166G4/C10/D1. Caspase 8 activation was particularly prominent after treatment with antibody 2161D2/C2, whereas caspase 3 activation was most strongly elicited by treatment with antibody 2172B12/C10.

Comprehensively, these sets of data highlight a differential ability of the different anti-NG2/CSPG4 antibodies to induce caspase-dependent apoptosis, which may tightly correlate with distinct isoform specificity of the antibodies and the documented differential expression of these putative isoforms on the different model cell lines. The findings further show that engagement of NG2/CSPG4 through the antibodies of this invention activates apoptotic events through the extrinsic pathway in the absence of canonical death receptor ligands.

Anti-NG2/CSPG4 antibodies induced programmed cell death through a dual pro-apoptotic and pro-autophagic action.

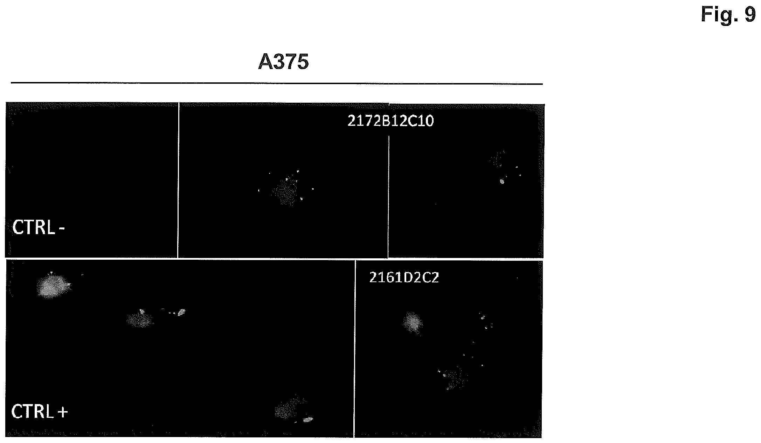

The possibility that the anti-NG2/CSPG4 antibodies of this invention could induce both caspase-dependent and autophagic programmed cell death was asserted by evaluating the expression of the two autophagic markers beclin-1 and LC3. To this end, A375 cells, were transiently transfected with a plasmid encoding the LC3-GFP fusion protein and treated with purified anti-NG2/CSPG4 antibodies as described above. As negative control we use untreated cells and as positive control cells treated with 100 nM Rapamicin for 24 and 48 hours. In untreated cells, LC3-GFP exhibited a diffuse cytoplasmic signal, whereas in antibody-treated cells undergoing autophagy, LC3-GFP chimeric proteins aggregated in autophagic vacuoles visualized as a punctuate cytoplasmic staining. Parallel autophagic evaluations were performed by flow cytometry using the Millipore FlowCellect FITC-LC3 Reporter Autophagy Assay kit. Although the signal appeared weak in all three cell lines (A375, Fig. 9A), HT1080NG2+ (Fig. 9B) and Hs578T (Fig. 9C) cells, a significant peak shift asserted that authophagic phenomena took place in the cells following antibody treatment. Antibody 2172D6/B1 appeared the most effective anti-NG2/CSPG4 antibody in inducing autophagic cell death, which was most pronounced in the melanoma and breast carcinoma cell lines, whereas antibodies 2172B12/C10 and 2166G4/C10/D1 gave quantitatively distinct effects of the A375 and Hs578T cells. Even in this case, differential induction of autophagy by the different antibodies in the different cell lines lend support to a differential involvement of different NG2/CSPG4 isoforms in the programmed cell death induced by the different antibodies. Described here below, but not part of the invention is the activation of autophagy in melanoma (A375) and soft-tissue sarcoma (HT1080NG2+) cell lines by anti-NG2/CSPG4 antibody treatment.

LC3 (microtubule-associated protein light chain 3), the most studied autophagy biomarker, was originally identified as a subunit of microtubule-associated proteins 1A and 1B (MAP1LC3) and was later found to contain similarity to yeast protein Apg8/Aut7/Cvt5. Distributed ubiquitously in eukaryotes, LC3 is expressed as 3 splice variants/isoforms (LC3A, LC3B and LC3C) which undergo post-translational processing, wherein, the unprocessed form of LC3 is proteolytically cleaved by Atg4 protease to form LC3-I with carboxyterminal exposed glycine. During autophagy, this exposed glycine of LC3-I is conjugated by Atg7 (an E1-like activity), Atg3 (an E2-like conjugating activity) and by Atg12-Atg5-Atg16L multimers (E3-like ligase activity) to phosphatidylethanolamine (PE) moiety for generating LC3-II. The lipophilic character of PE group facilitates LC3-II insertion into autophagosomes membranes, and as a result LC3-II is degraded when autophagosomes fuse with lysosomes to form autolysosomes for lysus of intra-autophagosomal components by lysosomal hydrolases. Conversion of LC3I to LC3II when correlated with autophagosome numbers is considered as the best marker of autophagy because LC3-II is the only well-characterized protein which specifically localize to autophagic structures throughout autophagy (from phagophore to lysosomal degradation).

Levels of LC3-I and LC3-II were measured by quantitative Western Blot in melanoma and fibrosarcoma cell lines, using a specific primary antibody that detect both bands ( -17 and -19 kDa), corresponding respectively to LC3-II and LC3-I.

In particular, cells were treated with the 4 anti-NG2/CSPG4-specific mAbs and a negative control mAb, total protein content was extracted and resolved by SDS-PAGE under reducing conditions, followed by transfer onto nitrocellulose membranes and immunoblotting with mAbs able to detect LC3I-II. β-Actin has been used as internal standard.

Microtuble-associated protein light chain 3 (LC3) has been used as a specific marker to monitor autophagy. Upon induction of autophagy, LC3 is conjugated to phosphatidylethanolamine and targeted to autophagic membranes. Therefore, changes in LC3 localization have been used to measure autophagy. A transfection assay was conducted using a plasmid encoding the

Autophagosome marker LC3 fused with the fluorescent protein EGFP. When GFP-LC3 is expressed in cultured cells, punctate signals are simply observed by fluorescence microscopy.

A375 and HT1080NG2+ cells, were transiently transfected with a plasmid encoding the LC3-GFP fusion protein and treated with purified anti-NG2/CSPG4 antibodies. As negative control we use untreated cells and as positive control cells treated with 100 nM Rapamicin for 24 and 48 hours. In untreated cells, LC3-GFP exhibited a diffuse cytoplasmic signal, whereas in antibody-treated cells undergoing autophagy, LC3-GFP chimeric proteins aggregated in autophagic vacuoles visualized as a punctuate cytoplasmic staining.

The results reveal that all antibodies induce endogenous LC3 conversion in each cell lines.

Moreover, autophagy induction in A375 and HT1080^{NG2+} cells was assessed by estimating the percentage GFP-LC3 expressing cells. As result, an evident activation of autophagic pathway was found in cell lines treated with the anti-NG2/CSPG4 mAbs.

## Claims

1. An antibody recognizing and binding to an antigen consisting of the ectodomain of the NG2/CSPG4 transmembrane proteoglycan, wherein said antibody induces cancer cell apoptosis upon binding to the antigen, said antibody having SEQ ID NO: 1 as VH and SEQ ID NO: 3 as VL, or SEQ ID NO: 5 as VH and SEQ ID NO: 7 as VL, or SEQ ID NO: 9 as VH and SEQ ID NO: 11 as VL, or SEQ ID NO: 13 as VH and SEQ ID NO: 15 as VL.

2. A composition comprising the antibody according to claim 1 and pharmaceutically acceptable excipients.

3. The antibody according to claim 1, wherein the antibody is embodied in
i) a bispecific antibody construct, or
ii) a fusion protein comprised of a therapeutic amount of the antibody and another therapeutic molecule, preferably wherein the other therapeutic molecule is a cytokine or an aptoptosis-inducing agent, or
iii) a CAR T (Chimeric Antigen Receptor T cell) construct in which an scFv of the antibody is expressed on the surface of a CAR T cell, such as to simultaneously engage NG2/CSPG4 expressing cancer cells and endogenous T cells and favor the maintenance of the CAR T cell in proximity to the cancer cell to trigger the activation of the engaged T cell.

4. The antibody according to claim 1 or the composition according to claim 2 for use as a medicament.

5. The antibody according to claim 1 or the composition according to claim 2 for use in the treatment of a cancer selected from the group consisting of: any variant, subtype or histotype of melanomas, soft-tissue, cartilage or bone sarcomas, head or neck carcinomas, colorectal carcinomas, lung carcinomas, prostate carcinomas, breast carcinomas, skin carcinomas, mesotheliomas, hematological neoplasia or Central Nervous System (CNS) and Peripheral Nervous System (PNS) tumors.

6. The antibody according to claim 1 or the composition according to claim 2 for use in the treatments and/or prevention of secondary metastatic and non-metastatic lesion formation, or relapsing local, loco-regional or distant tumor formation.

7. The antibody according to claim 1 or the composition according to claim 2 for use in the diagnosis of cancer.

## Patentansprüche

1. Antikörper, welcher ein Antigen, welches aus der Ektodomäne des NG2/CSPG4 transmembranen Proteoglykans besteht, erkennt und bindet, wobei der Antikörper nach Binden an das Antigen Krebszellen-Apoptose induziert, wobei der Antikörper SEQ ID NO: 1 als VH und SEQ ID NO: 3 als VL, oder SEQ ID NO: 5 als VH und SEQ ID NO: 7 als VL, oder SEQ ID NO: 9 als VH und SEQ ID NO: 11 als VL, oder SEQ ID NO: 13 als VH und SEQ ID NO: 15 als VL aufweist.

2. Zusammensetzung umfassend den Antikörper nach Anspruch 1 und pharmazeutisch akzeptable Trägerstoffe.

3. Antikörper nach Anspruch 1, wobei der Antikörper enthalten ist in
i) einem bispezifischem Antikörperkonstrukt, oder
ii) einem Fusionsprotein umfassend eine therapeutische Menge des Antikörpers und eines anderen therapeutischen Moleküls, wobei das andere therapeutische Molekül bevorzugt ein Zytokin oder ein Apoptose-induzierender Wirkstoff ist, oder
iii) ein CAR T (Chimäre Antigen-Rezeptor-T-Zelle)-Konstrukt, in welchem ein scFv des Antikörpers auf der Oberfläche einer CAR-T-Zelle exprimiert ist, um gleichzeitig NG2/CSPG4-exprimierende Krebszellen und endogene T-Zellen einzubinden und die Aufrechterhaltung der CAR T-Zelle in der Nähe der Krebszelle zu begünstigen, um die Aktivierung der eingebundenen T-Zelle auszulösen.

4. Antikörper nach Anspruch 1 oder Zusammensetzung nach Anspruch 2 zur Verwendung als ein Medikament.

5. Antikörper nach Anspruch 1 oder Zusammensetzung nach Anspruch 2 zur Verwendung in der Behandlung von Krebs ausgewählt aus der Gruppe bestehend aus: jeder Variante, Unterart oder Histoart von Melanomen, Weichgewebe, Knorpel- oder Knochensarkome, Kopf- oder Nackenkarzinome, kolorektale Karzinome, Lungenkarzinome, Prostatakarzinome, Brustkarzinome, Hautkarzinome, Mesotheliome, hämatologische Neoplasien oder Tumore im Zentralnervensystem (CNS) und peripheren Nervensystem (PNS).

6. Antikörper nach Anspruch 1 oder Zusammensetzung nach Anspruch 2 zur Verwendung in der Behandlung und/oder Prävention von sekundärer metastatischer und nicht-metastatischer Läsionsbildung, oder wiederkehrender lokaler, locoregionaler oder ferner Tumorbildung.

7. Antikörper nach Anspruch 1 oder Zusammensetzung nach Anspruch 2 zur Verwendung in der Krebsdiagnose.

## Revendications

1. Anticorps reconnaissant et se liant à un antigène consistant en l'ectodomaine du protéoglycane transmembranaire NG2/CSPG4, ledit anticorps induisant l'apoptose de cellules cancéreuses par liaison à l'antigène, ledit anticorps ayant la SEQ ID NO : 1 comme VH et la SEQ ID NO : 3 comme VL, ou la SEQ ID NO : 5 comme VH et SEQ ID NO : 7 comme VL, ou la SEQ ID NO : 9 comme VH et la SEQ ID NO : 11 comme VL, ou la SEQ ID NO : 13 comme VH et la SEQ ID NO : 15 comme VL.

2. Composition comprenant l'anticorps selon la revendication 1 et des excipients pharmaceutiquement acceptables.

3. Anticorps selon la revendication 1, dans lequel l'anticorps est incorporé dans
i) une construction d'anticorps bispécifique, ou
ii) une protéine de fusion contenant une quantité thérapeutique de l'anticorps et d'une autre molécule thérapeutique, dans laquelle l'autre molécule thérapeutique est de préférence une cytokine ou un agent induisant une apoptose, ou
iii) une construction CAR T (lymphocytes T à récepteur antigénique chimérique) dans laquelle un scFv de l'anticorps est exprimé sur la surface d'une cellule CAR T de manière à engager simultanément des cellules cancéreuses exprimant NG2/CSPG4 et des cellules T endogènes, et à favoriser le maintien de la cellule CAR T à proximité de la cellule cancéreuse pour activer la cellule T engagée.

4. Anticorps selon la revendication 1 ou composition selon la revendication 2 pour une utilisation comme médicament.

5. Anticorps selon la revendication 1 ou composition selon la revendication 2 pour une utilisation dans le traitement d'un cancer choisi dans le groupe constitué : de toute variante, sous-type ou histotype de mélanomes, de sarcomes des tissus mous, des cartilages ou des os, de carcinomes de la tête ou du cou, de carcinomes colorectaux, de carcinomes pulmonaires, de carcinomes de la prostate, de carcinomes du sein, de carcinomes de la peau, de mésothéliomes, de néoplasie hématologique ou de tumeurs du système nerveux central (SNC) et du système nerveux périphérique (SNP).

6. Anticorps selon la revendication 1 ou composition selon la revendication 2 pour une utilisation dans le traitement et/ou la prévention de la formation de lésions secondaires métastatiques et non métastatiques, ou de la formation récurrente de tumeurs locales, loco-régionales ou distantes.

7. Anticorps selon la revendication 1 ou composition selon la revendication 2 pour une utilisation dans le diagnostic du cancer.
